# EUROPÄISCHE PATENTSCHRIFT

(11) **EP 3 178 523 B1**
(45) Veröffentlichungstag und Bekanntmachung des Hinweises auf die Patenterteilung: **13.03.2019**
(21) Anmeldenummer: 16150576.3
(22) Anmeldetag: 08.01.2016
(51) Int. Cl.: A61Q 5/00, A61K 8/64, A61Q 5/12, A61K 8/899, A61K 8/9783

(54) **HAARBEHANDLUNGSMITTEL, INSBESONDERE HAARPFLEGEMITTEL, SOWIE DESSEN VERWENDUNG**
HAIR TREATMENT AGENT, IN PARTICULAR HAIR CARE AGENT, AND ITS USE
PRODUIT DE TRAITEMENT POUR CHEVEUX, PRODUIT DE SOIN POUR CHEVEUX ET LEUR UTILISATION

(30) Priorität: 08.12.2015 DE 102015015773
(43) Veröffentlichungstag der Anmeldung: 14.06.2017
(73) Patentinhaber: MAG Cosmetics GmbH, 74321 Bietigheim-Bissingen (DE)
(72) Erfinder: GRANZOW, Silke, 70771 Leinfelden (DE); LINK, Pascal, 70173 Stuttgart (DE)
(74) Vertreter: Von Rohr Patentanwälte Partnerschaft mbB

(56) Entgegenhaltungen:
- WO-A1-2005/116112
- WO-A2-2011/031706
- US-A1- 2013 149 271
- US-A1- 2014 261 518

## Beschreibung

Die vorliegende Erfindung betrifft das technische Gebiet der Kosmetik und insbesondere das technische Gebiet der Behandlung und/oder Pflege keratinischer Fasern oder Substrate, vorzugsweise menschlicher Haare.

Insbesondere betrifft die vorliegende Erfindung eine Zusammensetzung, insbesondere eine kosmetische Zusammensetzung, welche sich vorzugsweise zur Behandlung und/oder Pflege keratinischer Fasern oder Substrate, insbesondere menschlicher Haare, eignet, sowie deren Verwendung.

Des Weiteren betrifft die vorliegende Erfindung auch ein die erfindungsgemäße Zusammensetzung enthaltendes Haarbehandlungsmittel, insbesondere Haarpflegemittel, vorzugsweise in Form eines Haarfärbemittels, eines Haartönungsmittels, eines Haarwaschmittels, eines Shampoos, einer Haarspülung, eines Konditioniermittels (Conditioner), einer Haarcreme, einer Haarlotion, eines Haaröls, eines Haarwassers, eines Haargels, eines Haarwachses oder dergleichen.

Schließlich betrifft die vorliegende Erfindung eine Haarbehandlungseinheit, insbesondere zum Färben und/oder Tönen keratinischer Fasern oder Substrate, insbesondere menschlicher Haare, welche vorzugsweise in Form eines Sets (Kit-of-parts) vorliegt.

Menschliche Haare sind lange Hornfäden, welche im Wesentlichen aus Keratin gebildet werden (wobei Keratin einen Sammelbegriff für verschiedene wasserunlösliche Faserproteine darstellt, welche von Säugetieren gebildet werden und die Hornsubstanz charakterisieren, wobei man entsprechend ihrer molekularen Konformation als alpha-Helix oder beta-Faltblatt alpha-Keratine und beta-Keratine unterscheidet). Haare in diesem Sinne kommen nur bei Säugetieren vor, welche auf ihrer Haut zumindest teilweise Haare tragen (wohingegen die Schleimhäute stets unbehaart sind).

Es ist weiter bekannt, dass das Keratin in derartigen keratinischen Fasern bzw. Substraten, insbesondere menschlichen Haaren, durch Disulfidbrücken zwischen den Polypeptidketten in Form von Cystinresten stabilisiert wird. Wie nachfolgend noch im Detail geschildert, können exogene Faktoren, wie z. B. Hitze bzw. Wärme (Föhnen, Plätten, Glätten etc. der Haare), UV-Einstrahlung (z. B. Sonnenlicht), Chemikalien (z. B. Haarfärbemittel, Oxidationsmittel etc.) zur einer Schädigung dieser Haarstruktur, insbesondere unter Spaltung der Disulfidbrücken, führen. Derartige Oxidations-/ Reduktionsprozesse am Haar bilden aber auch die Grundlage für permanente Haarfärbe-, Haartönungs- und Haarblondierungsverfahren, des Weiteren auch für die Erzeugung von Dauerwellen etc.

Das Haar bzw. Haarorgan ist grob in drei Schichten aufgebaut: Cuticula, Cortex und Medulla. Die äußerste Schicht des Haars, welche auch Cuticula oder Schuppenschicht genannt wird, besteht aus flachen, übereinandergreifenden verhornten abgestorbenen Zellen, ähnlich zur Haarspitze orientiert wie bei einem Tannenzapfen. Sie besteht im Allgemeinen aus sechs bis zehn solcher Zell-Lagen. Die Schuppenschicht zeigt den Gesundheitszustand des Haares an. Beim gesunden Haar liegt die Schuppenschicht flach an und ergibt so eine glatte, durchscheinende Oberfläche. Das Licht wird optimal reflektiert und ergibt so den gesunden Glanz des Haares. Alkalisches Milieu öffnet die Schuppen, während saure Umgebung dieses verschließt. Die Cortex ("Rinde"), auch Faserschicht oder Faserstamm genannt, macht ca. 80 % des Haaranteils aus. Hier spielen sich alle für den Friseur relevanten chemischen Prozesse ab. Der Cortex besteht aus Faserbündeln, die aus einer großen Zahl feinster Keratinfasern, den Fibrillen, bestehen. Diese entstehen dadurch, dass sich Cortexzellen aneinanderlagern. Die Verbindung zwischen den beiden Zellen wird durch den Zellmembrankomplex hergestellt, welchen man sich als eine Art Kittsubstanz vorstellen kann. Die Reißfestigkeit und Elastizität des Haares sind auf diese Verkittung zurückzuführen. In wenigen Fällen, und dann auch nur bei dicken Haaren, fällt eine starke Auflockerung der Faserstruktur im Zentrum des Haares auf. In seiner Längsrichtung zeigt sich eine kanalförmig verlaufende, je nach Haardurchmesser unterschiedlich breit auftretende und unregelmäßig angeordnete Masse. Die im Faserstamm sonst so geordnete Struktur fehlt hier. Teilweise sind Hohlräume zu erkennen. Diesen Bereich des Haares nennt man den Markkanal oder einfach das Markmedulla. Für die berufliche Arbeit des Friseurs am, im und mit dem Kopfhaar hat die Medulla keinerlei Bedeutung.

Das menschliche Kopfhaar wird jeden Tag strapaziert und geschädigt: Beim Haarfärben und Haartönen, Blondieren, aber auch beim täglichen Haarewaschen, beim Styling, beim Glätten, beim Föhnen, bei Dauerwellen oder dergleichen werden die Haare strapaziert bzw. gestresst und wirken dadurch gealtert. Insbesondere wird die natürliche Schuppenschicht des Haares rauh und die Haare verlieren ihren Glanz, werden trocken und ausgefranst und schlechter kämmbar. Auch treten Schädigungen des natürlichen Haarkeratins ein; insbesondere werden natürliche Disulfidbrücken in der Keratin- bzw. Haarstruktur gespalten.

Insbesondere im Rahmen der Färbung oder Tönung der menschlichen Kopfhaare werden diese enorm strapaziert und geschädigt. Es wird geschätzt, dass ca. 40 % der erwachsenen Frauen in den Industrieländern Haarfärbemittel bzw. Haartönungsmittel benutzen. Eine Haarfärbung wird vorgenommen, um dem Haar andere Farbnuancen zu geben. Eine Haarfärbung ist meist auch noch nach mehrmaliger Haarwäsche mit tensidhaltigen Waschmitteln farbecht. Farbänderungen des Haares kann man durch Blondiermittel oder Haarfärbemittel erreichen. Die Haarfärbung lässt sich in Bezug auf die Anzahl der Haarwäschen nach der Färbung und der jeweiligen Haltbarkeit der Farbänderung in temporäre, semipermanente und permanente Färbungen einteilen. Eine temporäre Haarfärbung verschwindet nach dem ersten Waschen mit einem Haarshampoo; sie basiert auf einer physikalischen Haftung eines Farbstoffs. Eine semipermanente Haarfärbung hält ca. zwei bis zehn Haarwäschen aus. Eine permanente Färbung übersteht dagegen mehr als zehn Haarwäschen; hierbei werden kosmetische Farbstoffe chemisch an bestimmte Aminosäuren im Haar gekoppelt, und beim Blondieren werden Pigmente des Haares oxidiert.

Die temporäre Haarfärbung dient insbesondere dazu, die natürliche Haarfärbung leicht abzuändern. Diese Färbung lässt sich bei einmaliger Haarwäsche wieder vollständig entfernen. Für die temporäre Haarfärbung werden Azo-, Triphenylmethan- oder Anthrachinon-Farbstoffe verwendet. Zum Einsatz kommen die temporären Färbemittel üblicherweise als wässrige, alkoholische oder wässrigalkoholische Lösungen. Insbesondere in Verbindung mit Haarlacken können auch sehr kleine Glanzpigmente oder sogar phosphoreszierende Stoffe ins Haar eingebracht werden. Auch Wimpern- und Augenbrauenfärbemittel auf Basis sogenannter Mascara ("Wimperntusche") gehören zu den temporären Haarfärbemitteln. Als Farbmittel dienen zum Beispiel Ruß oder Ocker etc.

Bei der semipermanenten Haarfärbung verwendet man semipermanente Farbstoffe. Diese haben üblicherweise eine hohe Affinität zum Keratin des Haares, d. h. die Bindung der Farbstoffe erfolgt physikalisch. Als synthetische Haarfärbemittel werden insbesondere Nitrophenyldiamine, Azo- und Chinonimin-Farbstoffe in Verbindung mit organischen Lösungsvermittlern, wie Glykolethern oder Polypropylen, verwendet. Natürliche semipermanente Haarfärbemittel sind insbesondere Henna, Reng, Kamille, Holzextrakte, Rindenextrakte, Rastik etc.

Die permanente Haarfärbung ist mit Abstand die wichtigste Methode, um Haare zu färben. Eine permanente Haarfärbung ist praktisch nicht entfernbar; da das menschliche Haar im Monat jedoch etwa um 1 cm wächst, müssen Haarfärbungen etwa jeden Monat erneuert werden, sofern man eine gleichmäßige Haarfärbung beibehalten will.

Üblicherweise kommt eine Zweikomponenten-Haarfärbung zum Einsatz. Gelegentlich kommen auch Einkomponenten-Haarfarben auf Enzymbasis zum Einsatz, welche sich aber wegen der aufwendigeren Anwendung und geringerer Effizienz nicht in dieser Weise durchgesetzt haben.

Bei der permanenten Zweikomponenten-Haarfärbung (Oxidationshaarfärbung) werden Oxidationsbasen (d. h. leicht oxidierbare aromatische Verbindungen, wie z. B. Phenylendiamine, Toluylendiamine, Aminophenole etc.) zusammen mit sogenannten Nuancierern (d. h. Stoffen, welche den Farbton verändern) im Allgemeinen in Gegenwart von Wasserstoffperoxid und Ammoniak, Emulgatoren und Wasser auf das Haar aufgebracht: Die Farbstoffteilchen wandern dann in das durch das alkalische Medium aufgequollene Haarinnere und werden dort zu komplexen Farbstoffen oxidiert, wobei sie sich chemisch mit dem Haarkeratin verbinden. Erst durch die Oxidation der Farbstoffbasen durch das Wasserstoffperoxid entsteht der eigentliche Farbstoff. Daher bestehen käufliche permanente Haarfärbemittel üblicherweise aus zwei Komponenten, nämlich einer Komponente mit den Oxidationsbasen und den Nuancierern und einer weiteren Komponente mit Wasserstoffperoxid.

Neben den Farbstoffbasen enthält ein Haarfärbemittel üblicherweise noch Verdickungsmittel, wie beispielsweise Fettalkohole, Lanolin etc., so dass die Mischung cremiger wird. Damit die Oxidationsbasen nicht unter Lufteinfluss vorzeitig oxidiert werden, enthält ein Färbemittel üblicherweise ferner Antioxidantien, wie z. B. Natriumsulfit bzw. Natriumformaldehydsulfoxylat oder Ascorbinsäure. Ferner ist der sogenannten Farbpaste etwas Ammoniaklösung oder Monoethanolamin und üblicherweise auch etwas Tensid enthalten.

Zur Bereitstellung farbverändernder kosmetischer Mittel, insbesondere für die Haut oder für keratinhaltige Fasern bzw. Substrate, wie insbesondere menschliche Haare, kennt der Fachmann je nach Anforderungen an die Färbung diverse Färbesysteme. Für permanente, intensivere Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, welche unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, welche als färbende Komponente sogenannte direktziehende Farbstoffe ("Direktzieher") enthalten. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen keratinische Substrate oder Fasern, insbesondere menschliche Kopfhaare, aufgehellt oder gar gebleicht werden, werden die färbenden Farbstoffe meist oxidativ unter Einsatz von entsprechenden Oxidationsmitteln, wie Wasserstoffperoxid, entfärbt.

Um eine optimale Färbeleistung zu entfalten, benötigen oxidative Färbemittel im Allgemeinen einen alkalischen pH-Wert zur Ausfärbung, insbesondere in einem pH-Wertebereich zwischen etwa 9 und 11. Darüber hinaus beträgt die Anwendungsdauer für ansprechende Färbeergebnisse üblicherweise zwischen 10 und 45 Minuten. Es ist daher notwendig, dass das anwendungsbereite Färbemittel derart formuliert und konfektioniert ist, dass das Färbemittel sich einerseits gut auf den zu färbenden keratinischen Fasern verteilen lässt, andererseits jedoch in den zu färbenden Fasern während der Anwendungszeit verbleibt. Dazu ist es vorteilhaft, wenn das Färbemittel über eine bestimmte Viskosität verfügt, welche zwar das Auftragen des Mittels ermöglicht, jedoch das Mittel auch am Ort der Anwendung verbleiben lässt. Diese Viskosität kann durch polymere Verdickungsmittel im anwendungsbereiten Färbemittel eingestellt werden, wobei dieses Verdickungsmittel sowohl in der Farbveränderungszubereitung und/oder in der Oxidationsmittelzubereitung enthalten sein kann.

Um eine gute Mischung von Farbveränderungszubereitung und Oxidationsmittelzubereitung zu ermöglichen, ist es vorteilhaft, wenn die Farbveränderungszubereitung und Oxidationsmittelzubereitung über eine gute Fließfähigkeit verfügen. Daneben sollen die Oxidationsmittelzubereitungen auch über pflegende Eigenschaften verfügen und diese durch ein geeignetes optisches kosmetisches Erscheinungsbild der Haare verdeutlichen. Dazu sind insbesondere zweiphasige Oxidationsmittelzubereitungen geeignet, welche neben einer wässrigen oxidationsmittelhaltigen Phase eine weitere, pflegende hydrophobe Phase enthalten.

Derartige zweiphasige Oxidationsmittelzubereitungen mit pflegenden Eigenschaften sind beispielsweise aus der DE 10 2010 003 264 A1 bekannt. Darin werden zwei Phasen beschreiben, welche in Kontakt miteinander verpackt werden, wobei eine Phase eine wässrige Oxidationsmittelphase und eine weitere Phase eine hydrophobe Ölphase darstellt, wobei die Ölphase als Pflegeöle Carbonsäureester und/oder Paraffinöl enthält. Derartige Zusammensetzungen mögen zwar aufgrund der Anwesenheit der Ölphase dem geschädigten Haar einen gewissen Glanz zu verleihen, jedoch können geschädigte Haarstrukturen auf diese Weise nicht repariert werden. Auch der infolge der Schädigung und Strapazierung auftretenden schlechteren Kämmbarkeit und des verschlechterten Griffs des Haares wird hierdurch nicht in effizienter Weise entgegengewirkt.

Auch andere pflegende Zusätze, wie z. B. kationische Tenside, Keratine oder Alkylguanidin-Verbindungen etc., vermögen einer Schädigung des Haares nicht in effizienter Weise entgegenzutreten.

Darüber hinaus kommen zur Pflege geschädigter Haare auch sogenannte Silikone (synonym auch als Polysiloxane bezeichnet) zum Einsatz, welche durch eine physikalische Ummantelung des geschädigten Haares mit einer dünnen Silikonschicht eine künstliche glatte Oberfläche mit besserer Kämmbarkeit und besserem Glanz erzeugen sollen. Schädigungen des Haares können von rein silikonhaltigen Pflegemitteln aber lediglich überdeckt und nicht repariert werden. Da der tatsächliche Haarzustand des geschädigten Haares unter der Silikonschicht nicht mehr erkennbar ist, kann dies eine gezielte ausgleichende Pflege auf Dauer sogar erschweren. Weiterhin kann die Anwendung von Silikonen für die Haare problematisch werden, weil durch häufigen Gebrauch immer neue Silikonschichten aufgetragen werden, so dass ein sogenannter Build-up entstehen kann, welcher die Haare schwer und kraftlos werden lässt. Zudem können unter der Silikonschicht die geschädigten Haare unbemerkt weiter austrocknen, was zu verstärktem Spliss und vor allem Haarbruch führen kann.

Im Ergebnis sind aus dem Stand der Technik keine wirksamen oder verlässlichen Haarpflegeprodukte bekannt, welche universell und flexibel bei strapaziertem bzw. gestresstem und geschädigtem Haar anwendbar sind, wie es beispielsweise beim Färben oder Tönen, Blondieren, aber auch beim Stylen, Glätten, Föhnen, Dauerwellen etc. auftritt. Insbesondere vermögen die aus dem Stand der Technik bekannten Haarpflegemittel nicht in effizienter Weise das geschädigte Haar zu reparieren und gleichzeitig den optischen und haptischen Eindruck des geschädigten Haares zu verbessern.

Ansätze, zum Beispiel durch Färben, geschädigtes Haar zu reparieren wurden im Stand der Technik beschrieben, zum Beispiel in US 2013/0149271 A1.

Vor diesem Hintergrund besteht somit eine Aufgabe der vorliegenden Erfindung in der Bereitstellung einer Zusammensetzung, insbesondere einer kosmetischen Zusammensetzung, welche vorzugsweise zur Behandlung und/oder Pflege keratinischer Fasern oder Substrate, insbesondere menschlicher Haare, geeignet sein soll, wobei die zuvor geschilderten Nachteile des Standes der Technik zumindest weitgehend vermieden oder aber wenigstens abgeschwächt werden sollen.

Insbesondere soll die im Rahmen der vorliegenden Erfindung bereitzustellende Zusammensetzung eine verbesserte Pflege- und/oder Schutzfunktion in Bezug auf keratinische Fasern oder Substrate, insbesondere menschliche Haare, gegenüber mechanischen, physikalischen und/oder chemischen Beanspruchungen bereitstellen bzw. gewährleisten.

Eine weitere Aufgabe der vorliegenden Erfindung ist darin zu sehen, eine insbesondere zur Behandlung und/oder Pflege keratinischer Fasern oder Substrate, vorzugsweise menschlicher Haare, geeignete Zusammensetzung, insbesondere kosmetische Zusammensetzung, bereitzustellen, welche in ihren Anwendungs-, Handhabungs-, Pflege- und/oder Schutzeigenschaften in Bezug auf die zu behandelnden keratinischen Fasern oder Substrate, vorzugsweise menschliche Haare, im Vergleich zu entsprechenden Zusammensetzungen des Standes der Technik verbessert ist.

Eine nochmals weitere Aufgabe der vorliegenden Erfindung ist zudem darin zu sehen, eine solche insbesondere zur Behandlung und/oder Pflege keratinischer Fasern oder Substrate, vorzugsweise menschlicher Haare, geeignete Zusammensetzung, insbesondere kosmetische Zusammensetzung, bereitzustellen, bei welcher die Anwendungs-, Handhabungs-, Pflege- und/oder Schutzeigenschaften in Bezug auf die zu behandelnden keratinischen Fasern oder Substrate, vorzugsweise menschliche Haare, individuell eingestellt bzw. vor dem Hintergrund des jeweiligen Einsatzbereichs sozusagen maßgeschneidert werden können. Insbesondere soll die diesbezügliche Leistungsfähigkeit im Vergleich zu entsprechenden Zusammensetzungen des Standes der Technik weiterführend verbessert sein.

Zur Lösung des zuvor geschilderten Problems schlägt die vorliegende Erfindung somit - gemäß einem **ersten** Aspekt der vorliegenden Erfindung - eine Zusammensetzung, insbesondere eine kosmetische Zusammensetzung, vorzugsweise zur Behandlung und/oder Pflege keratinischer Fasern oder Substrate, insbesondere menschlicher Haare, gemäß Anspruch 1 vor; jeweils vorteilhafte Weiterbildungen und Ausgestaltungen dieses Erfindungsaspekts sind Gegenstand der entsprechenden Unteransprüche.

Weiterhin betrifft die vorliegende Erfindung - gemäß einem **zweiten** Aspekt der vorliegenden Erfindung - die erfindungsgemäße Verwendung der zuvor beschriebenen Zusammensetzung gemäß der vorliegenden Erfindung entsprechend dem diesbezüglichen unabhängigen Verwendungsanspruch; weitere, insbesondere vorteilhafte Ausgestaltungen und Weiterbildungen der erfindungsgemäßen Verwendung sind Gegenstand des diesbezüglichen Verwendungsunteranspruchs.

Darüber hinaus betrifft die vorliegende Erfindung - gemäß einem **dritten** Aspekt der vorliegenden Erfindung - auch ein die erfindungsgemäße Zusammensetzung enthaltendes Haarbehandlungsmittel, insbesondere Haarpflegemittel, vorzugsweise Haarfärbemittel, Haartönungsmittel, Haarwaschmittel, Shampoo, Haarspülung, Konditioniermittel (Conditioner), Haarcreme, Haarlotion, Haaröl, Haarwasser, Haargel, Haarwachs oder dergleichen, gemäß dem diesbezüglichen unabhängigen Anspruch.

Schließlich ist Gegenstand der vorliegenden Erfindung - gemäß einem **vierten** Aspekt der vorliegenden Erfindung - eine Haarbehandlungseinheit, insbesondere zum Färben und/oder Tönen keratinischer Fasern oder Substrate, insbesondere menschlicher Haare, vorzugsweise in Form eines Sets (Kit-of-parts), gemäß dem diesen erfindungsgemäßen Aspekt betreffenden unabhängigen Anspruch; weitere, jeweils vorteilhafte Weiterbildungen und Ausgestaltungen dieses Erfindungsaspekts sind Gegenstand des entsprechenden Unteranspruchs.

Es versteht sich von selbst, dass bei der nachfolgenden Beschreibung der vorliegenden Erfindung solche Ausgestaltungen, Ausführungsformen, Vorteile, Beispiele oder dergleichen, welche nachfolgend - zu Zwecken der Vermeidung unnötiger Wiederholungen - nur zu einem einzelnen Erfindungsaspekt ausgeführt werden, selbstverständlich auch in Bezug auf die übrigen Erfindungsaspekte entsprechend gelten, ohne dass es einer ausdrücklichen Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder aber anderenfalls mit dem Fachmann auf diesem Gebiet an sich geläufigen Bestimmungs- bzw. Messmethoden ermittelt bzw. bestimmt werden können. Sofern nicht anders angegeben, werden die zugrundeliegenden Werte bzw. Parameter unter Standardbedingungen (d. h. insbesondere bei einer Temperatur von 20 °C und/oder bei einem Druck von 1.013,25 hPa bzw. 1,01325 bar) ermittelt.

Im Übrigen gilt, dass bei sämtlichen nachstehend aufgeführten relativen bzw. prozentualen, insbesondere gewichtsbezogenen Mengenangaben zu beachten ist, dass diese Angaben im Rahmen der vorliegenden Erfindung vom Fachmann derart auszuwählen bzw. zu kombinieren sind, dass in der Summe - gegebenenfalls unter Einbeziehung weiterer Komponenten bzw. Inhaltsstoffe, insbesondere wie nachfolgend definiert - stets 100% bzw. 100 Gew.-% resultieren. Dies versteht sich für den Fachmann aber von selbst.

Dies vorausgeschickt, wird im Folgenden die vorliegende Erfindung näher beschrieben und erläutert. Im Zusammenhang mit der Beschreibung und Erläuterung der vorliegenden Erfindung werden auch weitergehende Vorteile, Eigenschaften und Merkmale der vorliegenden Erfindung aufgezeigt, welche jedoch in Bezug auf die vorliegende Erfindung keinesfalls beschränkend sind.

Gegenstand der vorliegenden Erfindung - gemäß einem **ersten** Aspekt der vorliegenden Erfindung - ist somit eine Zusammensetzung, insbesondere eine kosmetische Zusammensetzung, welche vorzugsweise zur Behandlung und/oder Pflege keratinischer Fasern oder Substrate, insbesondere menschlicher Haare, geeignet ist,
wobei die Zusammensetzung, insbesondere in Kombination und/oder Mischung,
(a) mindestens ein cystinhaltiges Polymer (nachfolgend auch als "Komponente (a)" bezeichnet), wobei das cystinhaltige Polymer und/oder die Komponente (a) ein Cystin/Siloxan-Copolymer (Cystin/Silikon-Copolymer) ist oder umfasst,
(b) mindestens ein Protein und/oder Peptid von *Pisum sativum L.* (nachfolgend auch als "Komponente (b)" bezeichnet), und
(c) mindestens einen Extrakt, von *Selaginella lepidophylla* (Unechte Rose von Jericho) (nachfolgend auch als "Komponente (c)" bezeichnet) enthält,
wobei die Zusammensetzung die Komponenten (a), (b) und (c) in einem gewichtsbezogenen Mengenverhältnis von Komponente (a) : Komponente (b) : Komponente (c) von 10-100 : 1-10 : 1-10 enthält.

Denn, wie die Anmelderin überraschend herausgefunden hat, eignet sich die erfindungsgemäße Zusammensetzung auf Basis einer ternären Kombination der drei zuvor definierten Komponenten (a), (b) und (c) in ausgezeichneter Weise zur Behandlung bzw. Pflege keratinischer Fasern oder Substrate, insbesondere menschlicher Haare.

Wie die Anmelderin in gleichermaßen vollkommen unerwarteter Weise herausgefunden hat, führt die ternäre Kombination der drei vorgenannten Komponenten (a), (b) und (c) - im Vergleich zu den einzelnen Komponenten oder einer binären Kombination von jeweils zwei der Komponenten - zu einem überraschenden synergistischen Effekt in Bezug auf die Behandlung bzw. Pflege keratinischer Fasern oder Substrate, insbesondere menschlicher Haare.

Insbesondere bewirkt die erfindungsgemäße Zusammensetzung bei der Behandlung keratinischer Fasern oder Substrate, insbesondere menschlicher Haare, dass strapazierte und/oder geschädigte keratinische Fasern bzw. Substrate, insbesondere menschliche Haare, einem natürlichen Reparaturmechanismus unterzogen werden: Das cystinhaltige Polymer der Komponente (a) und das Erbsenprotein bzw. -peptid der Komponente (b) führen zu einem natürlichen Reparaturmechanismus von geschädigtem Haar, insbesondere unter Ausbildung von neuen Disulfidbrücken in der geschädigten Keratinstruktur des Haars.

In diesem Zusammenhang führt das cystinhaltige Polymer der Komponente (a) dazu, dass kovalente Bindungen dieses Polymers mit dem Haarkeratin, insbesondere unter Ausbildung neuer disulfidischer Bindungen, ausgebildet werden, so dass die Komponente (a) an die Haarstruktur angebunden wird; dies führt neben dem vorgenannten Reparaturmechanismus auch dazu, dass ein langanhaltender Konditioniereffekt der Silikonkomponente des cystinhaltigen Polymers eintritt, da das Polymer kovalent an das Haarkeratin gebunden ist. Diese Wirkung wird durch das Erbsenprotein bzw. -peptid der Komponente (b) zusätzlich unterstützt.

Darüber hinaus bewirkt die Komponente (c) einen natürlichen Konditioniereffekt in der Art eines Silikons und unterstützt dabei die protektive Wirkung der übrigen Komponenten.

Im Ergebnis wirkt die erfindungsgemäße ternäre Zusammensetzung auf Basis der Kombination der vorgenannten Komponenten (a), (b) und (c) mechanischen und/oder physikalisch-chemischen Beanspruchungen keratinischer Fasern und Substrate, insbesondere menschlicher Haare, entgegen. Derartige Strapazierbelastungen treten beispielsweise beim Haarstylen, Haarföhnen, Haarglätten, Haarfärben, Haartönen etc. auf. Hier verhindert die erfindungsgemäße Zusammensetzung eine Schädigung des Haares bzw. minimiert diese Schädigung zumindest und wirkt den entsprechenden Beanspruchungen der vorgenannten Art in wirksamer Weise entgegen.

Auf diese Weise führt die erfindungsgemäße Zusammensetzung zu einem verbesserten, insbesondere weichen und/oder seidigen Griff des behandelten Haars, zu einer besseren Kämmbarkeit, zu einem verbesserten Glanz, zu einer verbesserten Festigkeit bei gleichzeitiger Weichheit und zu einer Verminderung von Sprödigkeit.

Geschädigte Keratinstrukturen werden unter Ausbildung neuer Disulfidbrücken in effizienter Weise entsprechend der ursprünglichen natürlichen Haarstruktur repariert.

Bei Behandlung des Haars mit der erfindungsgemäßen Zusammensetzung bildet sich in effizienter Weise eine protektive Hüllschicht um das strapazierte Haar aus, welche zu einer verbesserten Kämmbarkeit durch die glattere Oberfläche des behandelten Haars und zu einem verbesserten Glanz des behandelten Haars führt, wobei etwaige Haarschäden, wie insbesondere Spleißungen (Spliss) oder andere, in wirksamer Weise umschlossen werden.

Die erfindungsgemäße Zusammensetzung führt bei Behandlung von strapaziertem oder geschädigtem Haar also einerseits zur Ausbildung einer protektiven Hüllschicht und andererseits zu einer gleichzeitigen Reparatur unter Ausbildung neuer Disulfidbrücken.

Besonders vorteilhaft ist auch, dass die resultierende ternäre Kombination der drei vorgenannten Komponenten (a), (b) und (c) in besonderem Maße lagerstabil ist und zudem universell sowie flexibel verwendbar ist:
So kann die erfindungsgemäße Zusammensetzung beispielsweise als Haarbehandlungs- bzw. Haarpflegekomponente im Rahmen eines Haarfärbeverfahrens bzw. Haartönungsverfahrens eingesetzt werden, um im Rahmen der Haarfärbung oder Haartönung einer Schädigung des Haars entgegenzuwirken bzw. eine derartige Schädigung zu verhindern. Alternativ oder ergänzend kann aber die erfindungsgemäße Zusammensetzung auch in der Art eines Haarwaschmittels bzw. Shampoos oder eines Konditioniermittels (Conditioner) hergerichtet sein, welche beispielsweise nach einem Haarfärbe- oder Haartönungsverfahren angewendet werden.

Die erfindungsgemäße Zusammensetzung ist somit ein einzigartiges Haarbehandlungs- bzw. Haarpflegeprodukt, welches die Haare sowohl beim Färben, Tönen, Blondieren oder dergleichen als auch beim Stylen, Glätten, Föhnen etc. pflegt und schützt.

Auf der Grundlage ihrer Inhaltsstoffe kann die erfindungsgemäße Zusammensetzung, insbesondere unter Ausbildung neuer Disulfidbrücken, geschädigte Keratinstrukturen des Haars reparieren, die Schuppenschicht schützen und den Glanz der Haare erhöhen, so dass die Haare nach der Behandlung bzw. Pflege durch die erfindungsgemäße Zusammensetzung gesund wirken, weich und kräftig ausgebildet sind und einen hervorragenden Glanz zeigen, wobei darüber hinaus auch die Kämmbarkeit signifikant verbessert ist.

Dabei greift die erfindungsgemäße Zusammensetzung im Wesentlichen auf natürliche bzw. naturbasierte Inhaltsstoffe zurück: So enthält die Komponente (a) mit dem cystinhaltigen Polymer eine natürlich vorkommende Aminosäure, welche in Bezug auf eine Haarbehandlung folglich besonders kompatibel ist. Auch die Komponenten (b) und (c) sind naturbasiert bzw. Derivate natürlichen Ursprungs. Insgesamt ist daher die erfindungsgemäße Zusammensetzung im Hinblick auf eine Behandlung von menschlichen Haaren besonders kompatibel und darüber hinaus besonders haut- und umweltverträglich.

Wie zuvor ausgeführt, ist die erfindungsgemäße Zusammensetzung dennoch lagerstabil, insbesondere da die Komponenten (a), (b) und (c) untereinander nicht reaktiv ausgebildet sind, und in vielerlei Hinsicht universell und flexibel einsetzbar, insbesondere aufgrund der Kompatibilität der erfindungsgemäßen Zusammensetzung mit wässrigen, öligen und wässrig-öligen bzw. ölig-wässrigen Phasen. Infolgedessen ist die erfindungsgemäße Zusammensetzung auch im Hinblick auf übliche Zusammensetzungen zum Färben und Tönen der Haare kompatibel und kann in Kombination bzw. Mischung hiermit eingesetzt werden.

Im Ergebnis wird im Rahmen der Erfindung eine hocheffiziente, besonders wirksame und verträgliche Zusammensetzung, insbesondere kosmetische Zusammensetzung, zur Behandlung und Pflege von keratinischen Fasern oder Substraten, insbesondere menschlichen Haaren, bereitgestellt.

Insbesondere kann die Zusammensetzung nach der vorliegenden Erfindung ein Haarbehandlungsmittel, insbesondere ein Haarpflegemittel, vorzugsweise ein Haarfärbemittel, ein Haartönungsmittel, ein Haarwaschmittel, ein Shampoo, eine Haarspülung, ein Konditioniermittel (Conditioner), eine Haarcreme, eine Haarlotion, ein Haaröl, ein Haarwasser, ein Haargel, ein Haarwachs oder dergleichen, sein.

Mit anderen Worten kann die Zusammensetzung nach der vorliegenden Erfindung insbesondere als Haarbehandlungsmittel, insbesondere als Haarpflegemittel, vorzugsweise als Haarfärbemittel, Haartönungsmittel, Haarwaschmittel, Shampoo, Haarspülung, Konditioniermittel (Conditioner), Haarcreme, Haarlotion, Haaröl, Haarwasser, Haargel, Haarwachs oder dergleichen, ausgebildet und/oder hergerichtet sein oder Bestandteil hiervon sein.

Üblicherweise kann die Zusammensetzung außerdem mindestens einen kosmetisch kompatiblen Träger (Exzipienten) enthalten.

Insbesondere kann die Zusammensetzung nach der vorliegenden Erfindung die Inhaltsstoffe, insbesondere die Komponenten (a), (b) und (c) sowie gegebenenfalls vorhandene weitere Inhaltsstoffe, in einem kosmetisch kompatiblen Träger (Exzipienten) bzw. zusammen mit einem kosmetisch kompatiblen Träger (Exzipienten) enthalten.

Was das cystinhaltige Polymer und/oder die Komponente (a) der erfindungsgemäßen Zusammensetzung anbelangt, so kann das cystinhaltige Polymer und/oder die Komponente (a) ein gewichtsmittleres Molekulargewicht (Mw) im Bereich von 5.000 g/mol bis 1.000.000 g/mol, insbesondere im Bereich von 7.500 g/mol bis 750.000 g/mol, vorzugsweise im Bereich von 10.000 g/mol bis 500.000 g/mol, besonders bevorzugt im Bereich von 12.000 g/mol bis 100.000 g/mol, ganz besonders bevorzugt im Bereich von 15.000 g/mol bis 50.000 g/mol, noch mehr bevorzugt im Bereich von 17.500 g/mol bis 30.000 g/mol, aufweisen. Das gewichtsmittlere Molekulargewicht (Mw) kann dabei insbesondere mittels Gelpermeationschromatographie (GPC) bestimmt werden, insbesondere gemäß DIN 55671, vorzugsweise unter Verwendung eines Polystyrolstandards.

Was das cystinhaltige Polymer bzw. die Komponente (a) anbelangt, so ist es erfindungsgemäß diesbezüglich bevorzugt, wenn das cystinhaltige Polymer und/oder die Komponente (a) Cystin in gewichtsbezogenen Mengen, bezogen auf das cystinhaltige Polymer und/oder die Komponente (a), im Bereich von 10 bis 80 Gew.-%, insbesondere im Bereich von 15 bis 70 Gew.-%, vorzugsweise im Bereich von 20 bis 60 Gew.-%, besonders bevorzugt im Bereich von 25 bis 50 Gew.-%, enthält.

Bei der vorliegenden Erfindung ist oder umfasst das cystinhaltige Polymer und/oder die Komponente (a) ein Cystin/Siloxan-Copolymer (Cystin/Silikon-Copolymer).

Wie vorstehend ausgeführt, kann der Cystin-Bestandteil des Copolymers insbesondere eine Schädigung des betroffenen Haars entgegenwirken, insbesondere sozusagen über einen Reparaturmechanismus, vorzugsweise unter Ausbildung neuer Disulfidbrücken in der Keratinstruktur des Haars. Der Silikon-Bestandteil des Copolymers dagegen bildet eine protektive Hüllschicht um das strapazierte Haar aus, welche zu verbessertem Glanz, verbesserter Kämmbarkeit und einer glatteren Oberfläche des Haars führt. Durch die kovalente Anbindung, insbesondere über den Cystin-Bestandteil, unmittelbar an das Haar erfolgt eine dauerhafte Pflege bzw. Behandlung in Bezug auf das strapazierte oder geschädigte Haar.

Gemäß einer besonderen Ausführungsform der vorliegenden Erfindung kann es dabei vorgesehen sein, dass das cystinhaltige Polymer und/oder die Komponente (a) ein Copolymer umfasst oder ist, wobei das Copolymer eine oder mehrere Einheiten umfasst, wobei jede Einheit einen Cystinrest umfasst, wobei der Cystinrest jeweils an eine siliciumhaltige Komponente gebunden ist, wobei die siliciumhaltige Komponente mindestens eine Siloxanbindung umfasst.

Gemäß einer besonderen Ausführungsform der vorliegenden Erfindung kann es vorgesehen sein, dass das cystinhaltige Polymer und/oder die Komponente (a) ein Polymer ist oder umfasst, welches zwei oder mehr Einheiten der Formel [-B-A-B¹-] enthält, wobei der Rest A für HN-CH(COORₓ)CH₂-S-S-CH₂CH(COOR_{y})NR_{z} steht, wobei Rₓ und R_{y} gleich oder verschieden sind und jeweils für Wasserstoff oder eine Alkylgruppe stehen, R_{z} für Wasserstoff oder eine aminomodifizierende Gruppe steht und B und B¹ gleich oder verschieden sind und jeweils eine siliciumhaltige Gruppe darstellen, wobei die [-B-A-B¹-]-Einheiten durch Siloxanbindungen miteinander verbunden sind, wobei die Gruppe B in jeder [-B-A-B¹-]-Einheit gleich oder verschieden von der Gruppe B in einer anderen [-B-A-B¹-]-Einheit ist und die Gruppe B¹ in jeder [-B-A-B¹-]-Einheit gleich oder verschieden von der Gruppe B¹ in einer anderen [-B-A-B¹-]-Einheit ist und wobei eine Endgruppe des Polymers einen Rest aus der Gruppe von Cystin, Cystein, einem Bunte-Salz von Cystin, einem an einer oder beiden der Aminogruppen und/oder Carboxylgruppen chemisch modifizierten Cystin oder einem siliciumhaltigen Rest umfasst oder hieraus besteht. Auf diese Weise werden erfindungsgemäß besonders gute Ergebnisse erhalten.

Gemäß einer wiederum weiteren besonderen Ausführungsform der vorliegenden Erfindung kann es vorgesehen sein, dass das cystinhaltige Polymer und/oder die Komponente (a) ein Polymer ist oder umfasst, welches die folgende Struktur aufweist: 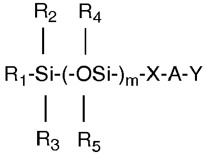 wobei der Rest X eine Verbindungsgruppe (Linker) darstellt, welche sich aus der Reaktion einer Aminogrupppe am Cystin mit einer reaktiven Aminogruppe an der siliciumhaltigen Gruppe ergibt, der Rest A für einen Rest der Formel HN-CH(COORₓ)CH₂-S-S-H₂CH(COOR_{y})NR_{z} steht, wobei Rₓ und R_{y} gleich oder verschieden sind und jeweils für Wasserstoff oder eine Alkylgruppe stehen und R_{z} für Wasserstoff oder eine aminomodifzierende Gruppe steht, und der Rest Y für H, eine aminomodifizierende Gruppe oder einen Rest der Formel 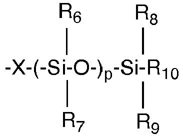 steht, R₁ bis R₁₀ gleich oder verschieden sind und jeweils für CH₃, OH oder Z stehen, wobei Z ein Rest einer der folgenden Formeln ist: 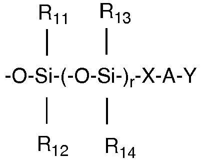 oder 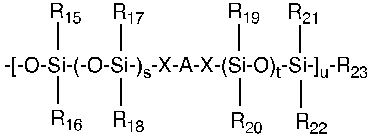 oder 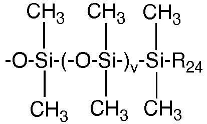 wobei R₁₁ bis R₂₂ gleich oder verschieden sind und jeweils für CH₃, OH oder Z stehen und R₂₃ und R₂₄ für CH₃ oder OH stehen, wobei m, p, r, s, t, u und v gleich oder verschieden sind und jeweils eine Zahl von 0 bis 100 einschließlich der Ober- und Untergrenzen darstellen, und
wobei mindestens eine der Reste R₁ bis R₁₀ für einen Rest Z steht, welcher einen Rest A enthält. Auch auf diese Weise werden besonders gute Ergebnisse erhalten.

Gemäß einer wiederum weiteren besonderen Ausführungsform der vorliegenden Erfindung kann es auch vorgesehen sein, dass das cystinhaltige Polymer und/oder die Komponente (a) ein Polymer ist oder umfasst, welches die folgende Struktur aufweist: 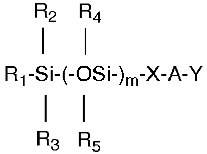 wobei der Rest X eine Verbindungsgruppe (Linker) der Formel (CH₂)₃-O-CH₂-CH(OH)CH₂ darstellt, der Rest A für einen Rest der Formel HN-CH(COORₓ)CH₂-S-S-H₂CH(COOR_{y})NR_{z} steht, wobei Rₓ und R_{y} gleich oder verschieden sind und jeweils für Wasserstoff oder eine Alkylgruppe stehen und R_{z} für Wasserstoff oder eine aminomodifzierende Gruppe steht, und der Rest Y für H, eine aminomodifizierende Gruppe oder einen Rest der Formel 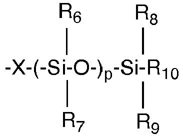 steht, R₁ bis R₁₀ gleich oder verschieden sind und jeweils für CH₃, OH oder Z stehen, wobei Z ein Rest einer der folgenden Formeln ist: 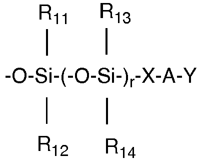 oder 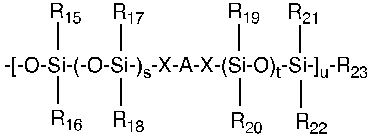 oder 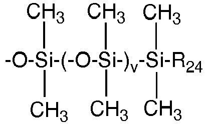 wobei R₁₁ bis R₂₂ gleich oder verschieden sind und jeweils für CH₃, OH oder Z stehen und R₂₃ und R₂₄ für CH₃ oder OH stehen, wobei m, p, r, s, t, u und v gleich oder verschieden sind und jeweils eine Zahl von 0 bis 100 einschließlich der Ober- und Untergrenzen darstellen, und
wobei mindestens eine der Reste R₁ bis R₁₀ für einen Rest Z steht, welcher einen Rest A enthält. Auch unter Verwendung dieses cystinhaltigen Polymers als Komponente (a) werden besonders gute Ergebnisse erhalten.

Gemäß einer wiederum weiteren besonderen Ausführungsform der vorliegenden Erfindung kann es vorgesehen sein, dass das cystinhaltige Polymer und/oder die Komponente (a) ein Polymer ist oder umfasst, welches die folgende Struktur aufweist:

HO-[Si(OH)₂-X-Cystin-X-Si(OH)₂-O]-H

wobei der Rest X eine Verbindungsgruppe (Linker) darstellt, insbesondere eine sich aus der Reaktion einer Aminogruppe am Cystin mit einer reaktiven Aminogruppe an der siliciumhaltigen Gruppe ergebende Verbindungsgruppe (Linker), vorzugsweise eine Verbindungsgruppe (Linker) der Formel (CH₂)₃-O-CH₂-CH(OH)CH₂, und wobei n eine Zahl von 1 bis 200 einschließlich der Ober- und Untergrenzen darstellt. Bei dieser Ausführungsform kann es insbesondere vorgesehen sein, dass das cystinhaltige Polymer Cystin in gewichtsbezogenen Mengen, bezogen auf das cystinhaltige Polymer, im Bereich von 10 bis 80 Gew.-%, insbesondere im Bereich von 15 bis 70 Gew.-%, vorzugsweise im Bereich von 20 bis 60 Gew.-%, besonders bevorzugt im Bereich von 25 bis 50 Gew.-%, enthält. Weiterhin kann es bei dieser Ausführungsform insbesondere vorgesehen sein, dass das cystinhaltige Polymer ein gewichtsmittleres Molekulargewicht (Mw) im Bereich von 5.000 g/mol bis 1.000.000 g/mol, insbesondere im Bereich von 7.500 g/mol bis 750.000 g/mol, vorzugsweise im Bereich von 10.000 g/mol bis 500.000 g/mol, besonders bevorzugt im Bereich von 12.000 g/mol bis 100.000 g/mol, ganz besonders bevorzugt im Bereich von 15.000 g/mol bis 50.000 g/mol, noch mehr bevorzugt im Bereich von 17.500 g/mol bis 30.000 g/mol, aufweist; wie zuvor ausgeführt, kann in diesem Zusammenhang das gewichtsmittlere Molekulargewicht (Mw) insbesondere mittels Gelpermeationschromatographie (GPC) bestimmt werden, insbesondere gemäß DIN 55671, vorzugsweise unter Verwendung eines Polystyrolstandards.

Gemäß einer ebenfalls weiteren besonderen Ausführungsform der vorliegenden Erfindung kann es vorgesehen sein, dass das cystinhaltige Polymer und/oder die Komponente (a) ein Cystin/Siloxan-Copolymer (Cystin/Silikon-Copolymer) vom Typ Poly[cystindiyl-bis-(polyglykol-propylsilantriol)] (synonym auch als Cystin-bis-pgpropylsilantriol bzw. als Poly(cystin-bis-pg-propylsilantriol) bezeichnet), insbesondere vom Typ Poly[cystindiyl-bis-(polyethylenglykol-propylsilantriol)] oder vom Typ Poly[cystindiyl-bis-(polypropylenglykol-propylsilantriol)], ist oder umfasst.

Die vorgenannten, im Rahmen der vorliegenden Erfindung als Komponente (a) verwendbaren cystinhaltigen Polymere sind kommerziell erhältliche Produkte, welche beispielsweise von der Croda Europe Ltd., East Yorkshire, Großbritannien, vertrieben werden (z. B. Crodasone Cystine).

Geeignete, im Rahmen als Komponente (a) verwendbare cystinhaltige Polymere sind beispielsweise auch in der EP 0 736 297 B1 oder in der DE 696 22 074 T2 beschrieben, deren gesamter diesbezüglicher Offenbarungsgehalt hiermit durch Bezugnahme eingeschlossen ist.

Üblicherweise können die vorgenannten cystinhaltigen Polymere, welche im Rahmen der vorliegenden Erfindung als Komponente (a) verwendet werden, hergestellt werden, indem man eine organofunktionelle siliciumhaltige Verbindung mit mindestens einer der Aminogruppen im Cystin oder in einem Cystinderivat reagieren lässt und anschließend das resultierende Reaktionsprodukt der siliciumhaltigen Verbindung des Cystins bzw. des Cystinderivats polymerisiert wird. Für weitergehende diesbezügliche Einzelheiten kann beispielsweise auf die EP 0 736 297 B1 oder die DE 696 22 074 T2 verwiesen werden.

Die Mengen an Komponente (a) in der erfindungsgemäßen Zusammensetzung können in weiten Bereichen variieren, insbesondere kann es vorgesehen sein, dass die Zusammensetzung das cystinhaltige Polymer und/oder die Komponente (a) in Mengen im Bereich von 0,01 bis 50 Gew.-%, insbesondere im Bereich von 0,05 bis 25 Gew.-%, vorzugsweise im Bereich von 0,1 bis 20 Gew.-%, besonders bevorzugt im Bereich von 0,5 bis 15 Gew.-%, noch mehr bevorzugt im Bereich von 1 bis 10 Gew.-%, bezogen auf die Zusammensetzung, enthält.

Dennoch kann anwendungsbezogen oder einzelfallbedingt von diesen Mengen abgewichen werden, ohne den Rahmen der vorliegenden Erfindung zu verlassen (was im Ermessen des Fachmanns liegt). Insbesondere wird der Fachmann die Menge in Abhängigkeit von der jeweiligen Zubereitungsform und ihre Anwendung wählen.

Was die Komponente (b) der erfindungsgemäßen Zusammensetzung anbelangt, so umfasst diese Komponente (b) -wie vorstehend ausgeführt-mindestens ein Protein und/oder Peptid von *Pisum sativum L..* In diesem Zusammenhang ist es bevorzugt, wenn die Zusammensetzung als Komponente (b) mindestens ein Peptid von *Pisum sativum L*., und/oder mindestens ein hydrolysiertes Protein von *Pisum sativum L.,* enthält. Insbesondere kann als Komponente (b) eine Di- und/oder Tripeptidfraktion verwendet werden, welche von einem Erbsenprotein ( *Pisum sativum L.*) durch Ultramembranfiltration erhalten werden kann.

Die Menge an Komponente (b) in der erfindungsgemäßen Zusammensetzung kann gleichermaßen in weiten Grenzen variieren. Insbesondere kann es vorgesehen sein, dass die Zusammensetzung nach der vorliegenden Erfindung das mindestens eine Protein und/oder Peptid aus der Pflanzengattung der Erbsen (*Pisum*) und/oder die Komponente (b) in Mengen im Bereich von 0,0001 bis 25 Gew.-%, insbesondere im Bereich von 0,001 bis 20 Gew.-%, vorzugsweise im Bereich von 0,005 bis 15 Gew.-%, besonders bevorzugt im Bereich von 0,01 bis 10 Gew.-%, noch mehr bevorzugt im Bereich von 0,02 bis 5 Gew.-%, bezogen auf die Zusammensetzung, enthält. Dennoch kann anwendungsbezogen oder einzelfallbedingt von diesen Mengen abgewichen werden, ohne den Rahmen der vorliegenden Erfindung zu verlassen (was im Ermessen des Fachmanns liegt). Insbesondere wird der Fachmann die Menge in Abhängigkeit von der jeweiligen Zubereitungsform und ihre Anwendung wählen.

Die vorgenannten, im Rahmen der vorliegenden Erfindung als Komponente (b) verwendbaren Wirk- bzw. Inhaltsstoffe sind ebenfalls kommerziell erhältliche Produkte, welche beispielsweise von der Active Concepts LLC, Lincolnton, USA, bzw. der Active Concepts LLC, Kaohsiung, Taiwan, vertrieben werden.

Was die Komponente (c) anbelangt, so enthält diese Komponente (c) - wie zuvor ausgeführt - mindestens einen Extrakt, von *Selaginella lepidophylla* (Unechte Rose von Jericho).

In diesem Zusammenhang ist es erfindungsgemäß bevorzugt, wenn die Zusammensetzung als Komponente (c) mindestens einen Extrakt von *Selaginella lepidophylla* (Unechte Rose von Jericho) enthält.

Auch die Menge an Komponente (c) in der erfindungsgemäßen Zusammensetzung kann in weiten Bereichen variieren. Insbesondere kann es vorgesehen sein, dass die Zusammensetzung den mindestens einen Inhaltsstoff, vorzugsweise den mindestens einen Extrakt, von *Selaginella lepidophylla* (Unechte Rose von Jericho) und/oder die Komponente (c) in Mengen im Bereich von 0,0001 bis 25 Gew.-%, insbesondere im Bereich von 0,001 bis 20 Gew.-%, vorzugsweise im Bereich von 0,005 bis 15 Gew.-%, besonders bevorzugt im Bereich von 0,01 bis 10 Gew.-%, noch mehr bevorzugt im Bereich von 0,02 bis 5 Gew.-%, bezogen auf die Zusammensetzung, enthält. Dennoch kann anwendungsbezogen oder einzelfallbedingt von diesen Mengen abgewichen werden, ohne den Rahmen der vorliegenden Erfindung zu verlassen (was im Ermessen des Fachmanns liegt). Insbesondere wird der Fachmann die Menge in Abhängigkeit von der jeweiligen Zubereitungsform und ihre Anwendung wählen.

Die vorgenannten, im Rahmen der vorliegenden Erfindung als Komponente (c) verwendbaren Wirk- bzw. Inhaltsstoffe sind gleichermaßen kommerziell erhältliche Produkte, welche beispielsweise von der Active Concepts LLC, Lincolnton, USA, bzw. der Active Concepts LLC, Kaohsiung, Taiwan, vertrieben werden.

Im Rahmen der vorliegenden Erfindung ist auch das gewichtsbezogene Mengenverhältnis der Komponenten (a), (b) und (c) von Bedeutung. In diesem Zusammenhang ist es bevorzugt, wenn die Zusammensetzung die Komponenten (a), (b) und (c) in einem gewichtsbezogenen Mengenverhältnis von Komponenten (a) : Komponente (b): Komponente (c) von 10-100 : 1-10 : 1-10, insbesondere von 10-50 : 1-10 : 1-10, enthält. Auf diese Weise werden besonders gute Ergebnisse enthalten.

Um eine besonders stabile, insbesondere lagerstabile, Zusammensetzung zu erhalten, ist es zudem von Vorteil, den pH-Wert der erfindungsgemäßen Zusammensetzung zu stabilisieren bzw. gezielt einzustellen (was im Allgemeinen mit den auf diesem Gebiet üblicherweise verwendeten pH-Stellmitteln bzw. pH-Puffersystemen erfolgt).

In diesem Zusammenhang ist es erfindungsgemäß bevorzugt, wenn die Zusammensetzung nach der vorliegenden Erfindung auf einen pH-Wert oberhalb von 5, insbesondere oberhalb von 5,5, eingestellt wird. Insbesondere kann dabei die erfindungsgemäße Zusammensetzung auf einen leicht sauren bis alkalischen pH-Wert eingestellt sein, insbesondere auf einen pH-Wert im Bereich von 5,5 bis 11,0.

Darüber hinaus kann die erfindungsgemäße Zusammensetzung auch noch weitere Inhaltsstoffe enthalten. Insbesondere kann es in diesem Zusammenhang vorgesehen sein, dass die Zusammensetzung außerdem (d) mindestens ein Radieschenwurzelfermentfiltrat, insbesondere ein Leuconostoc/Radieschenwurzelfermentfiltrat, insbesondere erhältlich durch Gärung von *Raphanus-sativus-Wurzeln* mittels des Mikroorganismus *Leuconostoc,* enthält ("Komponente (d)"). Die Komponente (d) wirkt im Rahmen der erfindungsgemäßen Zusammensetzung insbesondere antimikrobiell und/oder als Antischuppenwirkstoff und unterstützt auf diese Weise die pflegende Wirkung der erfindungsgemäßen Zusammensetzung.

Die Menge an der optionalen Komponente (d) bzw. des Radieschenwurzelfermentfiltrats kann gleichermaßen in weiten Grenzen variieren: insbesondere kann die erfindungsgemäße Zusammensetzung das Radieschenwurzelfermentfiltrat und/oder die Komponente (d) in Mengen im Bereich von 0,00001 bis 20 Gew.-%, insbesondere im Bereich von 0,0001 bis 10 Gew.-%, vorzugsweise im Bereich von 0,0005 bis 5 Gew.-%, besonders bevorzugt im Bereich von 0,001 bis 2 Gew.-%, bezogen auf die Zusammensetzung, enthalten. Dennoch kann anwendungsbezogen oder einzelfallbedingt von diesen Mengen abgewichen werden, ohne den Rahmen der vorliegenden Erfindung zu verlassen (was im Ermessen des Fachmanns liegt). Insbesondere wird der Fachmann die Menge in Abhängigkeit von der jeweiligen Zubereitungsform und ihre Anwendung wählen.

Weiterhin kann es vorgesehen sein, dass die Zusammensetzung nach der vorliegenden Erfindung außerdem (e) mindestens einen filmbildenden und/oder antistatischen Wirkstoff, insbesondere vom Quaternium- oder Polyquaternium-Typ, enthält ("Komponente (e)").

Die Menge an Komponente (e) in der erfindungsgemäßen Zusammensetzung kann gleichermaßen in weiten Bereichen variieren: Insbesondere kann es vorgesehen sein, dass die erfindungsgemäße Zusammensetzung den filmbildenden und/oder antistatischen Wirkstoff und/oder die Komponente (e) in Mengen im Bereich von 0,001 bis 40 Gew.-%, insbesondere im Bereich von 0,01 bis 30 Gew.-%, vorzugsweise im Bereich von 0,1 bis 25 Gew.-%, besonders bevorzugt im Bereich von 0,5 bis 20 Gew.-%, bezogen auf die Zusammensetzung, enthält. Dennoch kann anwendungsbezogen oder einzelfallbedingt von diesen Mengen abgewichen werden, ohne den Rahmen der vorliegenden Erfindung zu verlassen (was im Ermessen des Fachmanns liegt). Insbesondere wird der Fachmann die Menge in Abhängigkeit von der jeweiligen Zubereitungsform und ihre Anwendung wählen.

Weiterhin kann es erfindungsgemäß vorgesehen sein, dass die Zusammensetzung nach der vorliegenden Erfindung außerdem (f) mindestens ein Additiv und/oder mindestens einen Hilfsstoff ("Komponente (f)") enthält. Das Additiv und/oder der Hilfsstoff kann aber insbesondere ausgewählt sein aus der Gruppe von Verarbeitungshilfsstoffen, Stabilisatoren, Emulgatoren, Dispergatoren, Lösungsvermittlern, Lösemitteln, Konservierungsmitteln, Antioxidantien, Feuchthaltemitteln, Verdickungsmitteln, Viskositätsregulatoren, Antiseptika, Farbstoffen, Aromastoffen, Riechstoffen, Duftstoffen, Ölen, Parfümstoffen und Parfümölen, Streckmitteln, Bindemitteln, Netzmitteln, Vitaminen, Spurenelementen, Mineralstoffen, Mikronährstoffen, Pflegestoffen, pH-Stellmitteln und/oder pH-Wertpuffersystemen sowie deren Kombinationen.

Gemäß einer besonderes bevorzugten Ausführungsform der vorliegenden Erfindung betrifft die vorliegende Erfindung eine Zusammensetzung, insbesondere kosmetische Zusammensetzung, welche vorzugsweise zur Behandlung und/oder Pflege keratinischer Fasern oder Substrate, insbesondere menschlicher Haare, geeignet ist, insbesondere eine wie zuvor beschriebene Zusammensetzung nach der vorliegenden Erfindung,
wobei die Zusammensetzung, insbesondere in Kombination und/oder Mischung,
(a) mindestens ein cystinhaltiges Polymer ("Komponente (a)"), wobei das cystinhaltige Polymer und/oder die Komponente (a) ein Cystin/Siloxan-Copolymer (Cystin/Silikon-Copolymer) ist oder umfasst,
(b) mindestens ein Protein und/oder Peptid von *Pisum sativum L.* ("Komponente (b)"),
(c) mindestens einen Extrakt, von *Selaginella lepidophylla* (Unechte Rose von Jericho) ("Komponente (c)"),
(d) mindestens ein Radieschenwurzelfermentfiltrat, insbesondere ein Leuconostoc/Radieschenwurzelfermentfiltrat, insbesondere erhältlich durch Gärung von *Raphanus-sativus-Wurzeln* mittels des Mikroorganismus *Leuconostoc,* ("Komponente (d)"),
(e) mindestens einen filmbildenden und/oder antistatischen Wirkstoff, insbesondere vom Quaternium- oder Polyquaternium-Typ, ("Komponente (e)") und
(f) mindestens ein Additiv und/oder mindestens einen Hilfsstoff ("Komponente (f)"), insbesondere ausgewählt aus der Gruppe von Verarbeitungshilfsstoffen, Stabilisatoren, Emulgatoren, Dispergatoren, Lösungsvermittlern, Lösemitteln, Konservierungsmitteln, Antioxidantien, Feuchthaltemitteln, Verdickungsmitteln, Viskositätsregulatoren, Antiseptika, Farbstoffen, Aromastoffen, Riechstoffen, Duftstoffen, Ölen, Parfümstoffen und Parfümölen, Streckmitteln, Bindemitteln, Netzmitteln, Vitaminen, Spurenelementen, Mineralstoffen, Mikronährstoffen, Pflegestoffen, pH-Stellmitteln und/oder pH-Wertpuffersystemen sowie deren Kombinationen, enthält,
wobei die Zusammensetzung die Komponenten (a), (b) und (c) in einem gewichtsbezogenen Mengenverhältnis von Komponente (a) : Komponente (b) : Komponente (c) von 10-100 : 1-10 : 1-10 enthält.

Bezüglich der weitergehenden Einzelheiten zu dieser bevorzugten Ausführungsform kann auf die vorstehenden Ausführungen zu der erfindungsgemäßen Zusammensetzung Bezug genommen werden, welche auch in Bezug auf die bevorzugte Ausführungsform entsprechend gelten.

Weiterer Gegenstand der vorliegenden Erfindung - gemäß einem **zweiten** Aspekt der vorliegenden Erfindung - ist die Verwendung einer Zusammensetzung gemäß der vorliegenden Erfindung zur Behandlung und/oder Pflege keratinischer Fasern oder Substrate, insbesondere menschlicher Haare.

Insbesondere erfolgt die erfindungsgemäße Verwendung im Zusammenhang mit einem Haarfärbe- oder Haartönungsverfahren. Insbesondere kann die Zusammensetzung nach der vorliegenden Erfindung während und/oder nach der Haarfärbung oder Haartönung angewendet werden.

Insbesondere kann die erfindungsgemäße Zusammensetzung Verwendung finden zum Schutz keratinischer Fasern oder Substrate, insbesondere menschlicher Haare, vor exogenen Schädigungen, insbesondere oxidativen Schädigungen, insbesondere oxidativen Schädigungen durch Haarfärbe- oder Haartönungsmittel.

Des Weiteren kann die erfindungsgemäße Zusammensetzung Verwendung finden zur Verbesserung des Glanzes keratinischer Fasern oder Substrate, insbesondere menschlicher Haare, vorzugsweise zur Verbesserung des Glanzes gefärbter oder getönter keratinischer Fasern oder Substrate, insbesondere gefärbter oder getönter menschlicher Haare.

Weiterhin kann die erfindungsgemäße Zusammensetzung auch Verwendung finden zur Verbesserung der Waschbeständigkeit keratinischer Fasern oder Substrate, insbesondere menschlicher Haare, vorzugsweise zur Verbesserung der Waschbeständigkeit gefärbter oder getönter keratinischer Fasern oder Substrate, insbesondere gefärbter oder getönter menschlicher Haare.

Darüber hinaus kann die erfindungsgemäße Zusammensetzung Verwendung finden zur Verbesserung der Kämmbarkeit keratinischer Fasern oder Substrate, insbesondere menschlicher Haare, vorzugsweise zur Verbesserung der Kämmbarkeit gefärbter oder getönter keratinischer Fasern oder Substrate, insbesondere gefärbter oder getönter menschlicher Haare.

Ebenso kann die erfindungsgemäße Zusammensetzung Verwendung finden zur Verbesserung der statischen Eigenschaften keratinischer Fasern oder Substrate, insbesondere menschlicher Haare, vorzugsweise zur Verbesserung der statischen Eigenschaften gefärbter oder getönter keratinischer Fasern oder Substrate, insbesondere gefärbter oder getönter menschlicher Haare.

Auch kann die erfindungsgemäße Zusammensetzung Verwendung finden zur Verbesserung des Griffs und/oder der Weichheit keratinischer Fasern oder Substrate, insbesondere menschlicher Haare, vorzugsweise zur Verbesserung des Griffs und/oder der Weichheit gefärbter oder getönter keratinischer Fasern oder Substrate, insbesondere gefärbter oder getönter menschlicher Haare.

Schließlich kann die erfindungsgemäße Zusammensetzung auch Verwendung finden zur Verhinderung oder Verringerung der Schädigung keratinischer Fasern oder Substrate, insbesondere menschlicher Haare, bei (Haar-)Färbe- oder (Haar-)-Tönungsverfahren, insbesondere bei der oxidativen Farbveränderung.

Für weitergehende diesbezügliche Einzelheiten in Bezug auf die erfindungsgemäße Verwendung kann auf die vorstehenden Ausführungen zu der erfindungsgemäßen Zusammensetzung verwiesen werden, welche im Hinblick auf die erfindungsgemäße Verwendung entsprechend gelten.

Wiederum weiterer Aspekt der vorliegenden Erfindung - gemäß einem **dritten** Aspekt der vorliegenden Erfindung - ist ein Haarbehandlungsmittel, insbesondere Haarpflegemittel, vorzugsweise Haarfärbemittel, Haartönungsmittel, Haarwaschmittel, Shampoo, Haarspülung, Konditioniermittel (Conditioner), Haarcreme, Haarlotion, Haaröl, Haarwasser, Haargel, Haarwachs oder dergleichen, welches eine Zusammensetzung gemäß der vorliegenden Erfindung (wie zuvor beschrieben worden ist) enthält.

Für weitergehende diesbezügliche Einzelheiten zu diesem Erfindungsaspekt kann zu den vorstehenden Ausführungen zu den übrigen Erfindungsaspekten Bezug genommen werden, welche im Hinblick auf diesen Erfindungsaspekt entsprechend gelten.

Schließlich ist Gegenstand der vorliegenden Erfindung - gemäß einem **vierten** Aspekt der vorliegenden Erfindung - eine Haarbehandlungseinheit, insbesondere zum Färben und/oder Tönen keratinischer Fasern oder Substrate, insbesondere menschlicher Haare, vorzugsweise in Form eines Sets (Kit-of-parts),
wobei die Haarbehandlungseinheit als getrennte und/oder separate, insbesondere räumlich getrennte, aber funktional zusammenhängende und/oder funktional einander zugeordnete Komponenten aufweist:
(A) eine erste Komponente, welche mindestens einen Wirkstoff zum Färben und/oder Tönen keratinischer Fasern oder Substrate, insbesondere menschlicher Haare, enthält, einerseits; und
(B) eine zweite Komponente, welche eine Zusammensetzung gemäß einem der vorangehenden Ansprüche enthält, andererseits
aufweist.

Im Rahmen der erfindungsgemäßen Haarbehandlungseinheit kann es insbesondere vorgesehen sein, dass die erste Komponente (A) als Wirkstoff zum Färben und/oder Tönen keratinischer Fasern oder Substrate, insbesondere menschlicher Haare, mindestens einen Wirkstoff aus der Gruppe von Oxidationsfarbstoffen, Oxidationsfarbstoffvorprodukten, Oxidationsmitteln und direktziehenden Farbstoffen sowie deren Kombinationen, gegebenenfalls zusammen mit in Haarfärbemitteln üblichen Träger- und Hilfsstoffen, enthält.

Erfindungsgemäß kann es dabei vorgesehen sein, dass die Oxidationsfarbstoffvorprodukte mindestens eine Entwicklersubstanz und mindestens eine Kupplersubstanz umfassen.

Weiterhin kann es in diesem Zusammenhang erfindungsgemäß vorgesehen sein, dass das Oxidationsmittel ausgewählt ist aus der Gruppe von Wasserstoffperoxid, Peroxiden, Perborsäure und Perboraten sowie deren Kombinationen.

Üblicherweise ist es im Rahmen der erfindungsgemäßen Haarbehandlungseinheit vorgesehen, dass die erste Komponente (A) und die zweite Komponente (B) zur Mischung im Anwendungsfall, insbesondere im Rahmen eines Verfahrens zum Färben und/oder Tönen keratinischer Fasern oder Substrate, hergerichtet und/oder bestimmt sind.

Weiterhin kann es im Rahmen der erfindungsgemäßen Haarbehandlungseinheit vorgesehen sein, dass die erste Komponente (A) und die zweite Komponente (B) in einer gemeinsamen Umverpackung und/oder Verpackungseinheit, insbesondere in einer gemeinsamen Mehrkomponentenverpackungseinheit, zusammengeführt und/oder verpackt sind.

Dabei kann es erfindungsgemäß vorgesehen sein, dass die Umverpackung und/oder Verpackungseinheit, insbesondere die Mehrkomponentenverpackungseinheit, ein erstes, die erste Komponente (A) enthaltendes Behältnis (Container) sowie ein zweites, die zweite Komponente (B) enthaltendes Behältnis (Container) umfasst.

Weiterhin kann es bei dieser Ausführungsform erfindungsgemäß vorgesehen sein, dass die Umverpackung und/oder Verpackungseinheit, insbesondere die Mehrkomponentenverpackungseinheit, außerdem mindestens eine weitere Komponente (C) enthält. Die Komponente (C) kann insbesondere ausgewählt sein aus der Gruppe von (i) Applikationshilfsmitteln, wie Pinseln, Bürsten, Schwämmen, Kämmen oder dergleichen, (ii) Mischhilfsmitteln, insbesondere Anrührgefäßen und Misch- oder Rührhilfsmitteln, (iii) Schutzbekleidungsgegenständen, wie Handschuhen, insbesondere Einweg(schutz)handschuhen, (iv) Konditioniermitteln (Conditioner), (v) Haarwaschmitteln und Shampoos sowie (v) Instruktionen zur Handhabung, insbesondere Gebrauchsanweisungen und deren Kombinationen.

Für weitergehende diesbezügliche Einzelheiten in Bezug auf die erfindungsgemäße Haarbehandlungseinheit kann auf die vorstehenden Ausführungen zu den übrigen Erfindungsaspekten verwiesen werden, welche in Bezug auf die erfindungsgemäße Haarbehandlungseinheit entsprechend gelten.

Die vorliegende Erfindung wird anhand der nachfolgenden Ausführungsbeispiele veranschaulicht.

### AUSFÜHRUNGSBEISPIELE:

### 1. Herstellungsbeispiele

Nachfolgend werden verschiedene erfindungsgemäße Rezepturen und nichterfindungsgemäße Rezepturen (Vergleichsrezepturen) von Haarbehandlungs- bzw. Haarpflegemitteln hergestellt.

Die in den nachfolgenden Tabellen angegebenen Rezepturen werden jeweils durch Mischen der dort spezifizierten Ausgangs- bzw. Inhaltsstoffe unter dem Fachmann an sich bekannten Bedingungen und in an sich zu diesem Zweck bekannten Vorrichtungen hergestellt (z. B. unter Erwärmen, z. B. auf Temperaturen im Bereich von 30 bis 85 °C, beispielsweise in hierfür bekannten Vakuumprozessanlagen). Die jeweiligen Ausgangs- bzw. Inhaltsstoffe sind handelsüblich verfügbare Substanzen bzw. Chemikalien.

Als cystinhaltiges Polymer ("Komponente (a)") kommt bei den nachfolgenden Rezepturen, sofern dort vorhanden, ein Cystin/Siloxan-Copolymer (Cystin/Silikon-Copolymer) vom Typ Poly[cystindiyl-bis-(polyglykol-propyl-silantriol)] (INCI-Bezeichnung: Cystine Bis-PG-Propyl Silanetriol) der Croda Europe Ltd., East Yorkshire, Großbritannien, zum Einsatz (gewichtsmittleres Molekulargewicht Mw ca. 20.000; Cystinanteil in Bezug auf Copolymer ca. 33 %). Als "Komponente (b)" wird bei den nachfolgenden Rezepturen, sofern dort vorhanden, ein Peptid von *Pisum sativum L.* und als "Komponente (c)" ein Extrakt von *Selaginella lepidophylla* (Unechte Rose von Jericho) eingesetzt (jeweils vertrieben von der Active Concepts LLC, Lincolnton, USA, bzw. der Active Concepts LLC, Kaohsiung, Taiwan).

Die Rezepturen der Serie 1 können insbesondere als Haarpflegekomponente eines Haarfärbe- oder Haartönungsmittels (z. B. als Haarpflegekomponente im Rahmen einer entsprechenden Haarbehandlungseinheit zum Färben und/oder Tönen von Haaren bzw. eines entsprechenden Kit-of-parts) eingesetzt werden. Beispielsweise können die Rezepturen der Serie 1 beispielsweise einem üblichen Haarfärbemittel, einem üblichen Haartönungsmittel, einem üblichen Blondier- oder Aufhellungsmittel oder dergleichen während des entsprechenden Haarfärbe-, Haartönungs-, Blondierungs-, Aufhellungsprozesses etc. zugemischt werden, um das Haar während dieses Vorgangs zu schützen und zu pflegen. Die Rezeptur 1A ist erfindungsgemäß ausgebildet, wohingegen die Rezepturen 1B bis 1D nichterfindungsgemäß ausgebildet sind (Vergleichsrezepturen).

Die Rezeptur 2 kann insbesondere als konditionierende Haarpflegekomponente (Konditioniermittel bzw. Conditioner), als Haarkur, als Haarspülung etc. Verwendung finden (z. B. zur Anwendung nach einem Haarfärbe-, Haartönungs-, Blondierungs-, Aufhellungsprozesses etc., um das von Chemikalien freigespülte und gegebenenfalls gewaschene Haar weiterführend zu schützen und zu pflegen, aber auch für den alltäglichen Gebrauch, z. B. nach der Haarwäsche).

Die Rezeptur 3 schließlich kann gleichermaßen insbesondere als konditionierende Haarpflegekomponente (Konditioniermittel bzw. Conditioner), als Haarkur, als Haarspülung etc. zum Einsatz kommen (z. B. insbesondere zur Anwendung nach einem Haarfärbe-, Haartönungs-, Blondierungs-, Aufhellungsprozesses etc., aber auch für den alltäglichen Gebrauch, z. B. nach der Haarwäsche).

### Rezepturen der Serie 1

**Rezeptur 1A (erfindungsgemäß)**

| **Bestandteil** | **Gew.-%** |
|---|---|
| Cystinhaltiges Polymer ("Komponente (a)") | 6,25 |
| Peptid von *Pisum sativum L.* ("Komponente (b)") | 1,25 |
| Extrakt von *Selaginella lepidophylla* ("Komponente (c)") | 1,25 |
| Leuconostoc/Radieschenwurzelfermentfiltrat | 0,5 |
| Filmbildende und/oder antistatische Inhaltsstoffe (Polyquaternium-80) | 15,75 |
| Konservierungsmittel (Ethylhexylglycerin, Phenoxyethanol) | 0,60 |
| pH-Stellmittel (Natronlauge 10%ig, pH-Einstellung auf 8,2 - 8,5) | 5,50 |
| Wasser | **ad 100,00** |

**Rezeptur 1B (nichterfindungsgemäß)**

| **Bestandteil** | **Gew.-%** |
|---|---|
| Cystinhaltiges Polymer ("Komponente (a)") | ------ |
| Peptid von *Pisum sativum L.* ("Komponente (b)") | 1,25 |
| Extrakt von *Selaginella lepidophylla* ("Komponente (c)") | 1,25 |
| Leuconostoc/Radieschenwurzelfermentfiltrat | 0,5 |
| Filmbildende und/oder antistatische Inhaltsstoffe (Polyquaternium-80) | 15,75 |
| Konservierungsmittel (Ethylhexylglycerin, Phenoxyethanol) | 0,60 |
| pH-Stellmittel (Natronlauge 10%ig, pH-Einstellung auf 8,2 - 8,5) | 5,50 |
| Wasser | **ad 100,00** |

**Rezeptur 1C (nichterfindungsgemäß)**

| **Bestandteil** | **Gew.-%** |
|---|---|
| Cystinhaltiges Polymer ("Komponente (a)") | 6,25 |
| Peptid von *Pisum sativum L.* ("Komponente (b)") | ------ |
| Extrakt von *Selaginella lepidophylla* ("Komponente (c)") | 1,25 |
| Leuconostoc/Radieschenwurzelfermentfiltrat | 0,5 |
| Filmbildende und/oder antistatische Inhaltsstoffe (Polyquaternium-80) | 15,75 |
| Konservierungsmittel (Ethylhexylglycerin, Phenoxyethanol) | 0,60 |
| pH-Stellmittel (Natronlauge 10%ig, pH-Einstellung auf 8,2 - 8,5) | 5,50 |
| Wasser | **ad 100,00** |

**Rezeptur 1D (nichterfindungsgemäß)**

| **Bestandteil** | **Gew.-%** |
|---|---|
| Cystinhaltiges Polymer ("Komponente (a)") | 6,25 |
| Peptid von *Pisum sativum L.* ("Komponente (b)") | 1,25 |
| Extrakt von *Selaginella lepidophylla* ("Komponente (c)") | ------ |
| Leuconostoc/Radieschenwurzelfermentfiltrat | 0,5 |
| Filmbildende und/oder antistatische Inhaltsstoffe (Polyquaternium-80) | 15,75 |
| Konservierungsmittel (Ethylhexylglycerin, Phenoxyethanol) | 0,60 |
| pH-Stellmittel (Natronlauge 10%ig, pH-Einstellung auf 8,2 - 8,5) | 5,50 |
| Wasser | **ad 100,00** |

**Rezeptur 2 (erfindungsgemäß)**

| **Bestandteil** | **Gew.-%** |
|---|---|
| Cystinhaltiges Polymer ("Komponente (a)") | 0,38 |
| Peptid von *Pisum sativum L.* ("Komponente (b)") | 0,03 |
| Extrakt von *Selaginella lepidophylla* ("Komponente (c)") | 0,03 |
| Leuconostoc/Radieschenwurzelfermentfiltrat | 0,01 |
| Konservierungsmittel (Ethylhexylglycerin, Phenoxyethanol) | 1,00 |
| pH-Stellmittel (Citronensäure 50%ig) | 0,05 |
| Weitere Additive/Hilfsstoffe (Emulgator, Stabilisator, Pflegemittel, Feuchthaltemittel/Feuchtigkeitsspender, Viskositätsregler, filmbildende und/oder antistatische Inhaltsstoffe, Kämmbarkeitshilfen, Duftstoffe/Parfümöle) | ca. 19,00 |
| Wasser | **ad 100,00** |

**Rezeptur 3 (erfindungsgemäß)**

| **Bestandteil** | **Gew.-%** |
|---|---|
| Cystinhaltiges Polymer ("Komponente (a)") | 0,13 |
| Peptid von *Pisum sativum L.* ("Komponente (b)") | 0,03 |
| Extrakt von *Selaginella lepidophylla* ("Komponente (c)") | 0,03 |
| Leuconostoc/Radieschenwurzelfermentfiltrat | 0,01 |
| Konservierungsmittel (Ethylhexylglycerin, Phenoxyethanol) | 1,00 |
| pH-Stellmittel (Citronensäure 50%ig) | 0,05 |
| Weitere Additive/Hilfsstoffe (Emulgator, Stabilisator, Pflegemittel, Feuchthaltemittel/Feuchtigkeitsspender, Viskositätsregler, filmbildende und/oder antistatische Inhaltsstoffe, Kämmbarkeitshilfen, Duftstoffe/Parfümöle) | ca. 20,00 |
| Wasser | **ad 100,00** |

### 2. Anwendungstests zur verbesserten Nass- und Trockenkämmbarkeit

Zur Beurteilung der Kämmbarkeit (Nass- und Trockenkämmbarkeit) werden in sechs verschiedenen Ansätzen jeweils zwölf Haarsträhnen vom Typ Euro Naturhaar dunkelblond (Kerling International Haarfabrik GmbH, Backnang, Deutschland) mit einem handelsüblichen Haarfärbemittel (Blondiermasse) behandelt. In einem ersten Ansatz werden der Blondiermasse keine weiteren Zusätze beigemischt. In einem zweiten Ansatz wird der Blondiermasse eine hierfür vorgesehene handelsübliche Haarpflegekomponente auf Basis von Pflegeölen und Silikonen (Polysiloxanen) zugesetzt. In einem dritten bis sechsten Ansatz wird der Blondiermasse jeweils die Rezeptur 1A bzw. Rezeptur 1B bzw. Rezeptur 1C bzw. Rezeptur 1D zugesetzt. In einem siebten Ansatz wird der Blondiermasse ebenfalls die Rezeptur 1A zugesetzt.

Nach Anwendung des Färbemittels werden die Strähnen jeweils mit Wasser für zwei Minuten gespült und die wassernassen Strähnen zehnmal automatisch mit Hartgummikämmen gekämmt und die aufzuwendende Arbeit bestimmt. Bei dem siebten Ansatz werden die Wassernassen Strähnen vor dem Kämmen mit der erfindungsgemäßen Rezeptur 2 behandelt, gefolgt von einem weiteren Spülen der Strähnen mit Wasser für zwei Minuten. Es werden jeweils zwölf Haarsträhnen behandelt und gemessen. Als Wert für die Kämmbarkeit wird jeweils das arithmetische Mittel der aufzuwendenden Kämmarbeit gebildet und mit einer ganzzahligen Bewertungsskale von 0 bis 10 Bewertungspunkten korreliert (wobei ein Wert von 0 Bewertungspunkten einem Zustand "sehr schlecht" und ein Wert von 10 Bewertungspunkten einem Zustand "sehr gut" entspricht).

Die Ergebnisse, welche in der nachfolgenden Tabelle 1 wiedergegeben sind, zeigen eine deutliche Verbesserung der Nasskämmbarkeit durch die erfindungsgemäße Rezeptur 1A, was durch die zusätzliche Nachbehandlung mit der Rezeptur 2 noch weiterführend verbessert werden kann.

Überraschend ist dabei insbesondere, dass die erfindungsgemäße Rezeptur 1A mit einer ternären Kombination der vorgenannten Wirkkomponenten (a), (b) und (c) den jeweiligen nur binären Kombinationen der Vergleichsrezepturen 1B, 1C und 1D (und erst recht der im zweiten Ansatz verwendeten handelsüblichen Haarpflegekomponente) signifikant überlegen ist, was auf einen vollkommen unerwarteten synergistischen Effekt der ternären Wirkstoffkombination hindeutet.

**Tabelle 1: Nasskämmbarkeiten**

| **Ansatz** | **Bewertungspunkte** |
|---|---|
| Ansatz 1 (Vergleich) Blondiermasse ohne Zusatz | 0 |
| Ansatz 2 (Vergleich) Blondiermasse mit Öl-/Silikon-Zusatz aus Stand der Technik | 4 |
| Ansatz 3 (Erfindung) Blondiermasse mit Rezeptur 1A als Zusatz | 9 |
| Ansatz 4 (Vergleich) Blondiermasse mit Rezeptur 1B als Zusatz | 5 |
| Ansatz 5 (Vergleich) Blondiermasse mit Rezeptur 1C als Zusatz | 6 |
| Ansatz 6 (Vergleich) Blondiermasse mit Rezeptur 1D als Zusatz | 7 |
| Ansatz 7 (Erfindung) Blondiermasse mit Rezeptur 1A als Zusatz und Nachbehandlung mit Rezeptur 2 | 10 |

Zur Beurteilung der Trockenkämmbarkeit wird das zuvor geschilderte Vorgehen mit jeweils weiteren zwölf Haarsträhnen in weiteren sieben Ansätzen wiederholt, jedoch mit der Abweichung, dass die blondierten Strähnen nach dem zweiminütigen Spülen (d. h. vor dem Kämmen) für 2 Stunden luftgetrocknet werden, wobei nachfolgend die luftgetrockneten Strähnen zehnmal automatisch mit Hartgummikämmen gekämmt und die aufzuwendende Arbeit bestimmt wird.

Es werden gleichermaßen jeweils zwölf Haarsträhnen behandelt und gemessen. Als Wert für die Kämmbarkeit wird jeweils das arithmetische Mittel der aufzuwendenden Kämmarbeit gebildet und mit einer ganzzahligen Bewertungsskala von 0 bis 10 Bewertungspunkten korreliert (wobei ein Wert von 0 Bewertungspunkten einem Zustand "sehr schlecht" und ein Wert von 10 Bewertungspunkten einem Zustand "sehr gut" entspricht).

Auch diese Ergebnisse, welche in der nachfolgenden Tabelle 2 wiedergegeben sind, zeigen eine deutliche Verbesserung der Trockenkämmbarkeit durch die erfindungsgemäßen Haarpflegemittel, d. h. durch die erfindungsgemäße Rezeptur 1A, was durch die zusätzliche Nachbehandlung mit der Rezeptur 2 noch weiterführend verbessert werden kann.

Überraschend ist auch hierbei insbesondere die Tatsache, dass die erfindungsgemäße Rezeptur 1A mit einer ternären Kombination der vorgenannten Wirkkomponenten (a), (b) und (c) den jeweiligen nur binären Kombinationen der Vergleichsrezepturen 1B, 1C und 1D (und erst recht der im zweiten Ansatz verwendeten handelsüblichen Haarpflegekomponente) signifikant überlegen ist, was auch im Hinblick auf die Trockenkämmbarkeit auf einen vollkommen unerwarteten synergistischen Effekt der ternären Wirkstoffkombination hindeutet.

**Tabelle 2: Trockenkämmbarkeiten**

| **Ansatz** | **Bewertungspunkte** |
|---|---|
| Ansatz 1' (Vergleich) Blondiermasse ohne Zusatz | 0 |
| Ansatz 2' (Vergleich) Blondiermasse mit Öl-/Silikon-Zusatz aus Stand der Technik | 4 |
| Ansatz 3' (Erfindung) Blondiermasse mit Rezeptur 1A als Zusatz | 9 |
| Ansatz 4' (Vergleich) Blondiermasse mit Rezeptur 1B als Zusatz | 4 |
| Ansatz 5' (Vergleich) Blondiermasse mit Rezeptur 1C als Zusatz | 7 |
| Ansatz 6' (Vergleich) Blondiermasse mit Rezeptur 1D als Zusatz | 7 |
| Ansatz 7' (Erfindung) Blondiermasse mit Rezeptur 1A als Zusatz und Nachbehandlung mit Rezeptur 2 | 10 |

### 3. Weiterführende Anwendungstests: Kämmbarkeit, Glanz und Weichheit

Die erfindungsgemäße Haarpflegerezeptur 1A wird - sowohl in Abwesenheit von der erfindungsgemäßen Rezeptur 2 (nachfolgender Ansatz 3) und in Kombination mit der erfindungsgemäßen Rezeptur 2 (nachfolgender Ansatz 4)- im Rahmen einer Blondierungsbehandlung im Vergleich zu einer Blondierungsbehandlung ohne Pflegezusatz (nachfolgender Ansatz 1) und im Vergleich zu einer Blondierungsbehandlung mit herkömmlichem Pflegezusatz aus dem Stand der Technik (nachfolgend Ansatz 2) getestet.

Für die vier vorgenannten Versuchsansätze werden jeweils sechs Haarsträhnen vom Typ Euro Naturhaar dunkelblond (Kerling International Haarfabrik GmbH, Backnang, Deutschland) mit einem handelsüblichen Haarfärbemittel vom Typ Blondierungsmasse behandelt. Zu diesem Zweck wird die Blondierungsmasse entsprechend der Anwendungsempfehlung mit einem Pinsel in einer Färbeschale angerührt. Beim ersten Ansatz (Ansatz 1) erfolgt keine weitere Zugabe eines Haarpflegemittels zu der Blondierungsmasse, während beim zweiten Ansatz (Ansatz 2) ein herkömmlicher Pflegezusatz aus dem Stand der Technik (vgl. vorangehendes Beispiel 2) zu der Blondierungsmasse zugesetzt wird. Beim dritten und vierten Ansatz (Ansatz 3 und Ansatz 4) wird der Blondierungsmasse jeweils der erfindungsgemäße Pflegezusatz gemäß Rezeptur 1A zugesetzt. Die Zugabe des Pflegezusatzes in Ansatz 2, in Ansatz 3 und in Ansatz 4 erfolgt jeweils in die fertig gestellte Blondierungsmasse (d. h. nach dem Mischen von Färbemittel und Entwickler), es wird dann nochmals homogen verrührt. Die Einwirkzeit der Blondierungsmasse nach dem Einpinseln der Haarsträhnen beträgt jeweils 30 Minuten. Danach werden die Strähnen sorgfältig und rückstandsfrei mit VE-Wasser ausgewaschen. Lediglich bei Ansatz 4 werden für die Nachbehandlung mit der erfindungsgemäßen Rezeptur 2 die noch feuchten Strähnen verwendet: Die erfindungsgemäße Rezeptur 2 wird wie ein Konditioniermittel (Conditioner) in die Haare einmassiert, die Einwirkzeit beträgt 3 Minuten; danach wird wieder sorgfältig rückstandsfrei mit VE-Wasser ausgewaschen.

Alle auf die zuvor beschriebene Weise behandelten Strähnen der Ansätze 1 bis 4 werden jeweils geföhnt, bis keine Restfeuchte mehr zu spüren ist.

Alle Haarsträhnen werden im direkten Vergleich in den Punkten Kämmbarkeit (nass und trocken), Glanz und Haargefühl (Weichheit bzw. Griff) sensorisch und optisch von geschultem Laborpersonal beurteilt. Insgesamt werden auf diese Weise die verschieden behandelten Haarsträhnen miteinander verglichen und nach dem zuvor beschriebenen Bewertungssystem mit einer ganzzahligen Bewertungsskale von 0 bis 10 Bewertungspunkten bewertet (wobei ein Wert von 0 Bewertungspunkten einem Zustand "sehr schlecht" und ein Wert von 10 Bewertungspunkten einem Zustand "sehr gut" entspricht). Die Ergebnisse sind in der nachfolgenden Tabelle 3 wiedergegeben.

Die erfindungsgemäße Rezeptur 1A zeigt, vor allem in optionaler Kombinationsanwendung mit dem Konditioniermittel gemäß erfindungsgemäßer Rezeptur 2, in allen drei Kategorien, d. h. Kämmbarkeit (nass und trocken), Glanz und Haargefühl/Weichheit (Griff), einen äußerst positiven Einfluss. Diese Verbesserung ist sensorisch wie optisch wahrnehmbar.

Es werden noch weitere Haarfärbetests, wie Hellerfärbung und oxidative (permanente) Haarfärbung, an weiteren Haarsträhnen durchgeführt, bei denen sich die hervorragende Wirkung der erfindungsgemäßen Haarpflegezusammensetzungen weiter bestätigen lässt. Es lässt sich feststellen, dass der positive Einfluss der erfindungsgemäßen Haarpflegezusammensetzung bei einer Haarschädigung, wie sie insbesondere beim Blondieren auftritt, am signifikantesten ausgeprägt ist.

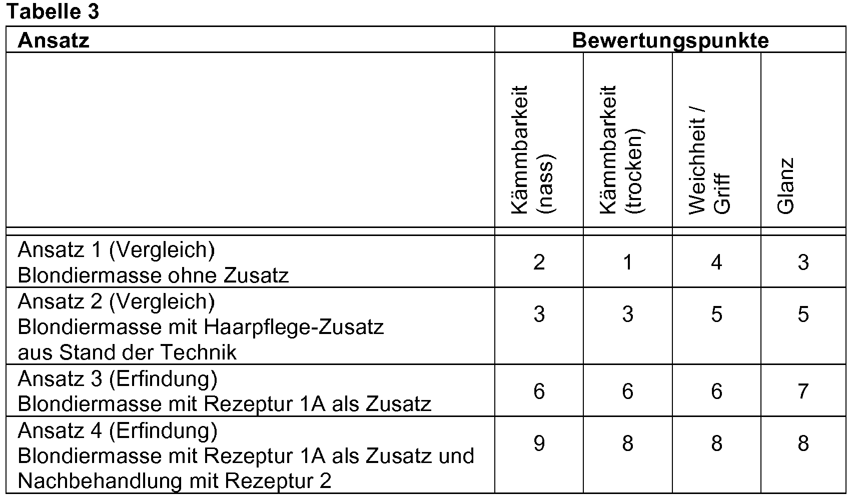

### 4. Weitere praktische Anwendungstests

Die erfindungsgemäßen Rezepturen 1A und 2 werden auch im Rahmen eines Probandentests, welcher durch drei Friseure und eine Friseurmeisterin durchgeführt wird, an 13 freiwilligen Personen untersucht, welche regelmäßig ihre Haare färben, blondieren und/oder wellen lassen. Es werden handelsübliche Oxidationsfarben und Blondierungen verwendet. Die detaillierten Ergebnisse liegen vor; grundsätzlich wird auch hier eine deutliche Verbesserung der relevanten Kriterien (d. h. insbesondere Kämmbarkeit, wie insbesondere Nass- und Trockenkämmbarkeit, Glanz, Griff und Weichheit) im Vergleich zu handelsüblichen Pflegeprodukten beobachtet.

## Patentansprüche

1. Zusammensetzung, insbesondere kosmetische Zusammensetzung, vorzugsweise zur Behandlung und/oder Pflege keratinischer Fasern oder Substrate, insbesondere menschlicher Haare,
wobei die Zusammensetzung, insbesondere in Kombination und/oder Mischung,
(a) mindestens ein cystinhaltiges Polymer ("Komponente (a)"), wobei das cystinhaltige Polymer und/oder die Komponente (a) ein Cystin/Siloxan-Copolymer (Cystin/Silikon-Copolymer) ist oder umfasst,
(b) mindestens ein Protein und/oder Peptid von *Pisum sativum L.* ("Komponente (b)"), und
(c) mindestens einen Extrakt von *Selaginella lepidophylla* (Unechte Rose von Jericho) ("Komponente (c)")
enthält,
wobei die Zusammensetzung die Komponenten (a), (b) und (c) in einem gewichtsbezogenen Mengenverhältnis von Komponente (a) : Komponente (b) : Komponente (c) von 10-100 : 1-10 : 1-10 enthält.

2. Zusammensetzung nach Anspruch 1,
wobei die Zusammensetzung ein Haarbehandlungsmittel, insbesondere ein Haarpflegemittel, vorzugsweise ein Haarfärbemittel, ein Haartönungsmittel, ein Haarwaschmittel, ein Shampoo, eine Haarspülung, ein Konditioniermittel (Conditioner), eine Haarcreme, eine Haarlotion, ein Haaröl, ein Haarwasser, ein Haargel, ein Haarwachs oder dergleichen, ist und/oder wobei die Zusammensetzung als Haarbehandlungsmittel, insbesondere als Haarpflegemittel, vorzugsweise als Haarfärbemittel, Haartönungsmittel, Haarwaschmittel, Shampoo, Haarspülung, Konditioniermittel (Conditioner), Haarcreme, Haarlotion, Haaröl, Haarwasser, Haargel, Haarwachs oder dergleichen, ausgebildet und/oder hergerichtet ist oder Bestandteil hiervon ist.

3. Zusammensetzung nach Anspruch 1 oder 2,
wobei die Zusammensetzung außerdem mindestens einen kosmetisch kompatiblen Träger (Exzipienten) enthält und/oder wobei die Zusammensetzung die Inhaltsstoffe, insbesondere die Komponenten (a), (b) und (c) sowie gegebenenfalls vorhandene weitere Inhaltsstoffe, in einem kosmetisch kompatiblen Träger (Exzipienten) bzw. zusammen mit einem kosmetisch kompatiblen Träger (Exzipienten) enthält.

4. Zusammensetzung nach einem der vorangehenden Ansprüche,
wobei das cystinhaltige Polymer und/oder die Komponente (a) ein gewichtsmittleres Molekulargewicht (Mw) im Bereich von 5.000 g/mol bis 1.000.000 g/mol, insbesondere im Bereich von 7.500 g/mol bis 750.000 g/mol, vorzugsweise im Bereich von 10.000 g/mol bis 500.000 g/mol, besonders bevorzugt im Bereich von 12.000 g/mol bis 100.000 g/mol, ganz besonders bevorzugt im Bereich von 15.000 g/mol bis 50.000 g/mol, noch mehr bevorzugt im Bereich von 17.500 g/mol bis 30.000 g/mol, aufweist; insbesondere wobei das gewichtsmittlere Molekulargewicht (Mw) mittels Gelpermeationschromatographie (GPC) bestimmt wird, insbesondere gemäß DIN 55671, vorzugsweise unter Verwendung eines Polystyrolstandards; und/oder
wobei das cystinhaltige Polymer und/oder die Komponente (a) Cystin in gewichtsbezogenen Mengen, bezogen auf das cystinhaltige Polymer und/oder die Komponente (a), im Bereich von 10 bis 80 Gew.-%, insbesondere im Bereich von 15 bis 70 Gew.-%, vorzugsweise im Bereich von 20 bis 60 Gew.-%, besonders bevorzugt im Bereich von 25 bis 50 Gew.-%, enthält; und/oder
wobei das cystinhaltige Polymer und/oder die Komponente (a) ein Copolymer umfasst oder ist, wobei das Copolymer eine oder mehrere Einheiten umfasst, wobei jede Einheit einen Cystinrest umfasst, wobei der Cystinrest jeweils an eine siliciumhaltige Komponente gebunden ist, wobei die siliciumhaltige Komponente mindestens eine Siloxanbindung umfasst; und/oder
wobei das cystinhaltige Polymer und/oder die Komponente (a) ein Cystin/Siloxan-Copolymer (Cystin/Silikon-Copolymer) vom Typ Poly[cystindiyl-bis-(polyglykol-propylsilantriol)], insbesondere vom Typ Poly[cystindiyl-bis-(polyethylenglykol-propylsilantriol)] oder vom Typ Poly[cystindiyl-bis-(polypropylenglykol-propylsilantriol)], ist oder umfasst.

5. Zusammensetzung nach einem der vorangehenden Ansprüche,
wobei das cystinhaltige Polymer und/oder die Komponente (a) ein Polymer ist oder umfasst, welches die folgende Struktur aufweist:
HO-[Si(OH)₂-X-Cystin-X-Si(OH)₂ -O]-H
wobei der Rest X eine Verbindungsgruppe (Linker) darstellt, insbesondere eine sich aus der Reaktion einer Aminogrupppe am Cystin mit einer reaktiven Aminogruppe an der siliciumhaltigen Gruppe ergebende Verbindungsgruppe (Linker), vorzugsweise eine Verbindungsgruppe (Linker) der Formel (CH₂)₃-O-CH₂-CH(OH)CH₂, und wobei n eine Zahl von 1 bis 200 einschließlich der Ober- und Untergrenzen darstellt;
insbesondere wobei das cystinhaltige Polymer Cystin in gewichtsbezogenen Mengen, bezogen auf das cystinhaltige Polymer, im Bereich von 10 bis 80 Gew.-%, insbesondere im Bereich von 15 bis 70 Gew.-%, vorzugsweise im Bereich von 20 bis 60 Gew.-%, besonders bevorzugt im Bereich von 25 bis 50 Gew.-%, enthält; und/oder
insbesondere wobei das cystinhaltige Polymer ein gewichtsmittleres Molekulargewicht (Mw) im Bereich von 5.000 g/mol bis 1.000.000 g/mol, insbesondere im Bereich von 7.500 g/mol bis 750.000 g/mol, vorzugsweise im Bereich von 10.000 g/mol bis 500.000 g/mol, besonders bevorzugt im Bereich von 12.000 g/mol bis 100.000 g/mol, ganz besonders bevorzugt im Bereich von 15.000 g/mol bis 50.000 g/mol, noch mehr bevorzugt im Bereich von 17.500 g/mol bis 30.000 g/mol, aufweist; insbesondere wobei das gewichtsmittlere Molekulargewicht (Mw) mittels Gelpermeationschromatographie (GPC) bestimmt wird, insbesondere gemäß DIN 55671, vorzugsweise unter Verwendung eines Polystyrolstandards.

6. Zusammensetzung nach einem der vorangehenden Ansprüche,
wobei die Zusammensetzung als Komponente (b) mindestens ein Peptid von *Pisum sativum L*., und/oder mindestens ein hydrolysiertes Protein von *Pisum sativum L.* enthält.

7. Zusammensetzung nach einem der vorangehenden Ansprüche,
wobei die Zusammensetzung das cystinhaltige Polymer und/oder die Komponente (a) in Mengen im Bereich von 0,01 bis 50 Gew.-%, insbesondere im Bereich von 0,05 bis 25 Gew.-%, vorzugsweise im Bereich von 0,1 bis 20 Gew.-%, besonders bevorzugt im Bereich von 0,5 bis 15 Gew.-%, noch mehr bevorzugt im Bereich von 1 bis 10 Gew.-%, bezogen auf die Zusammensetzung, enthält; und/oder
wobei die Zusammensetzung das mindestens eine Protein und/oder Peptid von *Pisum sativum* und/oder die Komponente (b) in Mengen im Bereich von 0,0001 bis 25 Gew.-%, insbesondere im Bereich von 0,001 bis 20 Gew.-%, vorzugsweise im Bereich von 0,005 bis 15 Gew.-%, besonders bevorzugt im Bereich von 0,01 bis 10 Gew.-%, noch mehr bevorzugt im Bereich von 0,02 bis 5 Gew.-%, bezogen auf die Zusammensetzung, enthält; und/oder
wobei die Zusammensetzung den mindestens einen Extrakt von *Selaginella lepidophylla* (Unechte Rose von Jericho) und/oder die Komponente (c) in Mengen im Bereich von 0,0001 bis 25 Gew.-%, insbesondere im Bereich von 0,001 bis 20 Gew.-%, vorzugsweise im Bereich von 0,005 bis 15 Gew.-%, besonders bevorzugt im Bereich von 0,01 bis 10 Gew.-%, noch mehr bevorzugt im Bereich von 0,02 bis 5 Gew.-%, bezogen auf die Zusammensetzung, enthält.

8. Zusammensetzung nach einem der vorangehenden Ansprüche,
wobei die Zusammensetzung auf einen pH-Wert oberhalb von 5, insbesondere oberhalb von 5,5, eingestellt ist und/oder wobei die Zusammensetzung auf einen leicht sauren bis alkalischen pH-Wert eingestellt ist, insbesondere auf einen pH-Wert im Bereich von 5,5 bis 11,0.

9. Zusammensetzung nach einem der vorangehenden Ansprüche,
wobei die Zusammensetzung außerdem (d) mindestens ein Radieschenwurzelfermentfiltrat, insbesondere ein Leuconostoc/Radieschenwurzelfermentfiltrat, insbesondere erhältlich durch Gärung von *Raphanus-sativus-Wurzeln* mittels des Mikroorganismus *Leuconostoc,* enthält ("Komponente (d)"); insbesondere wobei die Zusammensetzung das Radieschenwurzelfermentfiltrat und/oder die Komponente (d) in Mengen im Bereich von 0,00001 bis 20 Gew.-%, insbesondere im Bereich von 0,0001 bis 10 Gew.-%, vorzugsweise im Bereich von 0,0005 bis 5 Gew.-%, besonders bevorzugt im Bereich von 0,001 bis 2 Gew.-%, bezogen auf die Zusammensetzung, enthält; und/oder
wobei die Zusammensetzung außerdem (e) mindestens einen filmbildenden und/oder antistatischen Wirkstoff, insbesondere vom Quaternium- oder Polyquaternium-Typ, enthält ("Komponente (e)"); insbesondere wobei die Zusammensetzung den filmbildenden und/oder antistatischen Wirkstoff und/oder die Komponente (e) in Mengen im Bereich von 0,001 bis 40 Gew.-%, insbesondere im Bereich von 0,01 bis 30 Gew.-%, vorzugsweise im Bereich von 0,1 bis 25 Gew.-%, besonders bevorzugt im Bereich von 0,5 bis 20 Gew.-%, bezogen auf die Zusammensetzung, enthält; und/oder
wobei die Zusammensetzung außerdem (f) mindestens ein Additiv und/oder mindestens einen Hilfsstoff ("Komponente (f)") enthält, insbesondere ausgewählt aus der Gruppe von Verarbeitungshilfsstoffen, Stabilisatoren, Emulgatoren, Dispergatoren, Lösungsvermittlern, Lösemitteln, Konservierungsmitteln, Antioxidantien, Feuchthaltemitteln, Verdickungsmitteln, Viskositätsregulatoren, Antiseptika, Farbstoffen, Aromastoffen, Riechstoffen, Duftstoffen, Ölen, Parfümstoffen und Parfümölen, Streckmitteln, Bindemitteln, Netzmitteln, Vitaminen, Spurenelementen, Mineralstoffen, Mikronährstoffen, Pflegestoffen, pH-Stellmitteln und/oder pH-Wertpuffersystemen sowie deren Kombinationen.

10. Zusammensetzung, insbesondere kosmetische Zusammensetzung, vorzugsweise zur Behandlung und/oder Pflege keratinischer Fasern oder Substrate, insbesondere menschlicher Haare, insbesondere Zusammensetzung nach einem der vorangehenden Ansprüche,
wobei die Zusammensetzung, insbesondere in Kombination und/oder Mischung,
(a) mindestens ein cystinhaltiges Polymer ("Komponente (a)"), wobei das cystinhaltige Polymer und/oder die Komponente (a) ein Cystin/Siloxan-Copolymer (Cystin/Silikon-Copolymer) ist oder umfasst,
(b) mindestens ein Protein und/oder Peptid von *Pisum sativum L.* ("Komponente (b)"),
(c) mindestens einen Extrakt von *Selaginella lepidophylla* (Unechte Rose von Jericho) ("Komponente (c)"),
(d) mindestens ein Radieschenwurzelfermentfiltrat, insbesondere ein Leuconostoc/Radieschenwurzelfermentfiltrat, insbesondere erhältlich durch Gärung von *Raphanus-sativus-Wurzeln* mittels des Mikroorganismus *Leuconostoc,* ("Komponente (d)"),
(e) mindestens einen filmbildenden und/oder antistatischen Wirkstoff, insbesondere vom Quaternium- oder Polyquaternium-Typ, ("Komponente (e)") und
(f) mindestens ein Additiv und/oder mindestens einen Hilfsstoff ("Komponente (f)"), insbesondere ausgewählt aus der Gruppe von Verarbeitungshilfsstoffen, Stabilisatoren, Emulgatoren, Dispergatoren, Lösungsvermittlern, Lösemitteln, Konservierungsmitteln, Antioxidantien, Feuchthaltemitteln, Verdickungsmitteln, Viskositätsregulatoren, Antiseptika, Farbstoffen, Aromastoffen, Riechstoffen, Duftstoffen, Ölen, Parfümstoffen und Parfümölen, Streckmitteln, Bindemitteln, Netzmitteln, Vitaminen, Spurenelementen, Mineralstoffen, Mikronährstoffen, Pflegestoffen, pH-Stellmitteln und/oder pH-Wertpuffersystemen sowie deren Kombinationen,
enthält,
wobei die Zusammensetzung die Komponente (a), (b) und (c) in einem gewichtsbezogenen Mengenverhältnis von Komponenten (a) : Komponente (b) : Komponente (c) von 10-100 : 1-10 : 1-10 enthält.

11. Verwendung einer Zusammensetzung gemäß einem der vorangehenden Ansprüche zur Behandlung und/oder Pflege keratinischer Fasern oder Substrate, insbesondere menschlicher Haare.

12. Verwendung nach Anspruch 11
im Zusammenhang mit einem Haarfärbe- oder Haartönungsverfahren, insbesondere wobei die Zusammensetzung während und/oder nach der Haarfärbung oder Haartönung angewendet wird; oder aber
zum Schutz keratinischer Fasern oder Substrate, insbesondere menschlicher Haare, vor exogenen Schädigungen, insbesondere oxidativen Schädigungen, insbesondere oxidativen Schädigungen durch Haarfärbe- oder Haartönungsmittel; oder aber
zur Verbesserung des Glanzes keratinischer Fasern oder Substrate, insbesondere menschlicher Haare, vorzugsweise zur Verbesserung des Glanzes gefärbter oder getönter keratinischer Fasern oder Substrate, insbesondere gefärbter oder getönter menschlicher Haare; oder
zur Verbesserung der Waschbeständigkeit keratinischer Fasern oder Substrate, insbesondere menschlicher Haare, vorzugsweise zur Verbesserung der Waschbeständigkeit gefärbter oder getönter keratinischer Fasern oder Substrate, insbesondere gefärbter oder getönter menschlicher Haare; oder aber
zur Verbesserung der Kämmbarkeit keratinischer Fasern oder Substrate, insbesondere menschlicher Haare, vorzugsweise zur Verbesserung der Kämmbarkeit gefärbter oder getönter keratinischer Fasern oder Substrate, insbesondere gefärbter oder getönter menschlicher Haare; oder aber
zur Verbesserung der statischen Eigenschaften keratinischer Fasern oder Substrate, insbesondere menschlicher Haare, vorzugsweise zur Verbesserung der statischen Eigenschaften gefärbter oder getönter keratinischer Fasern oder Substrate, insbesondere gefärbter oder getönter menschlicher Haare; oder aber
zur Verbesserung des Griffs und/oder der Weichheit keratinischer Fasern oder Substrate, insbesondere menschlicher Haare, vorzugsweise zur Verbesserung des Griffs und/oder der Weichheit gefärbter oder getönter keratinischer Fasern oder Substrate, insbesondere gefärbter oder getönter menschlicher Haare; oder aber zur Verhinderung oder Verringerung der Schädigung keratinischer Fasern oder Substrate, insbesondere menschlicher Haare, bei (Haar-)Färbe- oder (Haar-)Tönungsverfahren, insbesondere bei der oxidativen Farbveränderung.

13. Haarbehandlungsmittel, insbesondere Haarpflegemittel, vorzugsweise Haarfärbemittel, Haartönungsmittel, Haarwaschmittel, Shampoo, Haarspülung, Konditioniermittel (Conditioner), Haarcreme, Haarlotion, Haaröl, Haarwasser, Haargel, Haarwachs oder dergleichen, enthaltend eine Zusammensetzung gemäß einem der vorangehenden Ansprüche.

14. Haarbehandlungseinheit, insbesondere zum Färben und/oder Tönen keratinischer Fasern oder Substrate, insbesondere menschlicher Haare, vorzugsweise in Form eines Sets (Kit-of-parts),
wobei die Haarbehandlungseinheit als getrennte und/oder separate, insbesondere räumlich getrennte, aber funktional zusammenhängende und/oder funktional einander zugeordnete Komponenten aufweist:
(A) eine erste Komponente, welche mindestens einen Wirkstoff zum Färben und/oder Tönen keratinischer Fasern oder Substrate, insbesondere menschlicher Haare, enthält, einerseits; und
(B) eine zweite Komponente, welche eine Zusammensetzung gemäß einem der vorangehenden Ansprüche enthält, andererseits
aufweist.

15. Haarbehandlungseinheit nach Anspruch 14,
wobei die erste Komponente (A) als Wirkstoff zum Färben und/oder Tönen keratinischer Fasern oder Substrate, insbesondere menschlicher Haare, mindestens einen Wirkstoff aus der Gruppe von Oxidationsfarbstoffen, Oxidationsfarbstoffvorprodukten, Oxidationsmitteln und direktziehenden Farbstoffen sowie deren Kombinationen, gegebenenfalls zusammen mit in Haarfärbemitteln üblichen Träger- und Hilfsstoffen, enthält; und/oder
wobei die Oxidationsfarbstoffvorprodukte mindestens eine Entwicklersubstanz und mindestens eine Kupplersubstanz umfassen; und/oder
wobei das Oxidationsmittel ausgewählt ist aus der Gruppe von Wasserstoffperoxid, Peroxiden, Perborsäure und Perboraten sowie deren Kombinationen; und/oder
wobei die erste Komponente (A) und die zweite Komponente (B) zur Mischung im Anwendungsfall, insbesondere im Rahmen eines Verfahrens zum Färben und/oder Tönen keratinischer Fasern oder Substrate, hergerichtet und/oder bestimmt sind; und/oder
wobei die erste Komponente (A) und die zweite Komponente (B) in einer gemeinsamen Umverpackung und/oder Verpackungseinheit, insbesondere in einer gemeinsamen Mehrkomponentenverpackungseinheit, zusammengeführt und/oder verpackt sind; insbesondere wobei die Umverpackung und/oder Verpackungseinheit, insbesondere die Mehrkomponentenverpackungseinheit, ein erstes, die erste Komponente (A) enthaltendes Behältnis (Container) sowie ein zweites, die zweite Komponente (B) enthaltendes Behältnis (Container) umfasst; und/oder insbesondere wobei die Umverpackung und/oder Verpackungseinheit, insbesondere die Mehrkomponentenverpackungseinheit, außerdem mindestens eine weitere Komponente (C) enthält, insbesondere ausgewählt aus der Gruppe von (i) Applikationshilfsmitteln, wie Pinseln, Bürsten, Schwämmen, Kämmen oder dergleichen, (ii) Mischhilfsmitteln, insbesondere Anrührgefäßen und Misch- oder Rührhilfsmitteln, (iii) Schutzbekleidungsgegenständen, wie Handschuhen, insbesondere Einweg(schutz)handschuhen, (iv) Konditioniermitteln (Conditioner), (v) Haarwaschmitteln und Shampoos sowie (v) Instruktionen zur Handhabung, insbesondere Gebrauchsanweisungen und deren Kombinationen.

## Claims

1. A composition, in particular a cosmetic composition, preferably for the treatment and/or care of keratinous fibres or substrates, in particular human hair,
wherein the composition contains, in particular in combination and/or as a mixture,
(a) at least one cystine-containing polymer ("component (a)"), wherein the cystine-containing polymer and/or the component (a) is or comprises a cystine/siloxane copolymer (cystine/silicone copolymer),
(b) at least one protein and/or peptide from *Pisum sativum L.* ("component (b)"), and
(c) at least one extract of *Selaginella lepidophylla* (false rose of Jericho) ("component (c)")
wherein the composition contains the components (a), (b) and (c) in a proportion by weight of component (a) : component (b) : component (c) of 10-100 : 1-10 : 1-10.

2. The composition as claimed in claim 1,
wherein the composition is a hair treatment agent, in particular a hair care agent, preferably a hair colouring agent, a hair tinting agent, a hair washing agent, a shampoo, a hair rinse, a conditioner, a hair cream, a hair lotion, a hair oil, a hair tonic, a hair gel, a hair wax or the like, and/or wherein the composition is formulated and/or manufactured as a hair treatment agent, in particular as a hair care agent, preferably as a hair colouring agent, hair tinting agent, hair washing agent, shampoo, hair rinse, conditioner, hair cream, hair lotion, hair oil, hair tonic, hair gel, hair wax or the like, or is a component thereof.

3. The composition as claimed in claim 1 or claim 2,
wherein the composition furthermore contains at least one cosmetically compatible excipient and/or wherein the composition contains the ingredients, in particular the components (a), (b) and (c) as well as further ingredients where appropriate, in a cosmetically compatible excipient or together with a cosmetically compatible excipient.

4. The composition as claimed in one of the preceding claims,
wherein the cystine-containing polymer and/or the component (a) has a mass average molecular weight (Mw) in the range 5000 g/mol to 1000000 g/mol, in particular in the range 7500 g/mol to 750000 g/mol, preferably in the range 10000 g/mol to 500000 g/mol, particularly preferably in the range 12000 g/mol to 100000 g/mol, more particularly preferably in the range 15000 g/mol to 50000 g/mol, yet more preferably in the range 17500 g/mol to 30000 g/mol; in particular wherein the mass average molecular weight (Mw) is determined by means of gel permeation chromatography (GPC), in particular in accordance with DIN 55671, preferably using a polystyrene standard; and/or
wherein the cystine-containing polymer and/or the component (a) contains cystine in quantities by weight, with respect to the cystine-containing polymer and/or the component (a), in the range 10 to 80% by weight, in particular in the range 15 to 70% by weight, preferably in the range 20 to 60% by weight, particularly preferably in the range 25 to 50% by weight; and/or
wherein the cystine-containing polymer and/or the component (a) comprises or is a copolymer, wherein the copolymer comprises one or more units, wherein each unit comprises a cystine residue, wherein the cystine residue is respectively bonded to a silicon-containing component, wherein the silicon-containing component comprises at least one siloxane compound; and/or
wherein the cystine-containing polymer and/or the component (a) is or comprises a cystine/siloxane copolymer (cystine/silicone copolymer) of the poly[cystine bis-polyglycol-propyl silanetriol] type, in particular of the poly[cystine bis-polyethylene glycol-propyl silanetriol)] type or of the poly[cystine bis-polypropylene glycol-propyl silanetriol] type.

5. The composition as claimed in one of the preceding claims,
wherein the cystine-containing polymer and/or the component (a) is or comprises a polymer which has the following structure:
HO-[Si(OH)₂-X-cystine-X-Si(OH)₂-O]-H
wherein the residue X represents a linker, in particular a linker obtained by the reaction of an amino group on the cystine with a reactive amino group on the silicon-containing group, preferably a linker with the formula (CH₂)₃-O-CH₂-CH(OH)CH₂, and wherein n is a number from 1 to 200 including the upper and lower limits;
in particular wherein the cystine-containing polymer contains cystine in quantities by weight, with respect to the cystine-containing polymer, in the range 10 to 80% by weight, in particular in the range 15 to 70% by weight, preferably in the range 20 to 60% by weight, particularly preferably in the range 25 to 50% by weight; and/or
in particular wherein the cystine-containing polymer has a mass average molecular weight (Mw) in the range 5000 g/mol to 1000000 g/mol, in particular in the range 7500 g/mol to 750000 g/mol, preferably in the range 10000 g/mol to 500000 g/mol, particularly preferably in the range 12000 g/mol to 100000 g/mol, more particularly preferably in the range 15000 g/mol to 50000 g/mol, yet more preferably in the range 17500 g/mol to 30000 g/mol; in particular wherein the mass average molecular weight (Mw) is determined by means of gel permeation chromatography (GPC), in particular in accordance with DIN 55671, preferably using a polystyrene standard.

6. The composition as claimed in one of the preceding claims,
wherein the composition contains, as component (b), at least one peptide from *Pisum sativum L.,* and/or at least one hydrolysed protein from *Pisum sativum L..*

7. The composition as claimed in one of the preceding claims,
wherein the composition contains the cystine-containing polymer and/or the component (a) in quantities in the range 0.01 to 50% by weight, in particular in the range 0.05 to 25% by weight, preferably in the range 0.1 to 20% by weight, particularly preferably in the range 0.5 to 15% by weight, yet more preferably in the range 1 to 10% by weight, with respect to the composition; and/or
wherein the composition contains the at least one protein and/or peptide from *Pisum sativum* and/or the component (b) in quantities in the range 0.0001 to 25% by weight, in particular in the range 0.001 to 20% by weight, preferably in the range 0.005 to 15% by weight, particularly preferably in the range 0.01 to 10% by weight, yet more preferably in the range 0.02 to 5% by weight, with respect to the composition; and/or
wherein the composition contains the at least one extract of *Selaginella lepidophylla* (false rose of Jericho) and/or the component (c) in quantities in the range 0.0001 to 25% by weight, in particular in the range 0.001 to 20% by weight, preferably in the range 0.005 to 15% by weight, particularly preferably in the range 0.01 to 10% by weight, yet more preferably in the range 0.02 to 5% by weight, with respect to the composition.

8. The composition as claimed in one of the preceding claims,
wherein the composition is adjusted to a pH of more than 5, in particular more than 5.5, and/or wherein the composition is adjusted to a slightly acidic to alkaline pH, in particular to a pH in the range 5.5 to 11.0.

9. The composition as claimed in one of the preceding claims,
wherein the composition further contains (d) at least one radish root fermentation filtrate, in particular a leuconostoc/radish root fermentation filtrate, in particular obtainable by fermentation of *Raphanus-sativus* roots using the microorganism *Leuconostoc* ("component (d)"); in particular wherein the composition contains the radish root fermentation filtrate and/or the component (d) in quantities in the range 0.00001 to 20% by weight, in particular in the range 0.0001 to 10% by weight, preferably in the range 0.0005 to 5% by weight, particularly preferably in the range 0.001 to 2% by weight, with respect to the composition; and/or
wherein the composition further contains (e) at least one film-forming and/or antistatic substance, in particular of the quaternium or polyquaternium type ("component (e)"); in particular wherein the composition contains the film-forming and/or antistatic substance and/or the component (e) in quantities in the range 0.001 to 40% by weight, in particular in the range 0.01 to 30% by weight, preferably in the range 0.1 to 25% by weight, particularly preferably in the range 0.5 to 20% by weight, with respect to the composition; and/or
wherein the composition furthermore contains (f) at least one additive and/or at least one auxiliary substance ("component (f)"), in particular selected from the group formed by processing aids, stabilizers, emulsifying agents, dispersants, solubilizers, solvents, preservatives, antioxidants, humectants, thickening agents, viscosity regulators, antiseptics, colorants, flavourings, aromatic substances, fragrances, oils, perfumes and perfume oils, extenders, binders, wetting agents, vitamins, trace elements, minerals, micronutrients, care products, pH regulators and/or pH buffering systems, as well as combinations thereof.

10. A composition, in particular a cosmetic composition, preferably for the treatment and/or care of keratinous fibres or substrates, in particular human hair, in particular the composition as claimed in one of the preceding claims,
wherein the composition contains, in particular in combination and/or as a mixture,
(a) at least one cystine-containing polymer ("component (a)"), wherein the cystine-containing polymer and/or the component (a) is or comprises a cystine/siloxane copolymer (cystine/silicone copolymer),
(b) at least one protein and/or peptide from *Pisum sativum L.* ("component (b)"),
(c) at least one extract of *Selaginella lepidophylla* (false rose of Jericho) ("component (c)"),
(d) at least one radish root fermentation filtrate, in particular a leuconostoc/radish root fermentation filtrate, in particular obtainable by fermentation of *Raphanus-sativus* roots using the microorganism *Leuconostoc,* ("component (d)"),
(e) at least one film-forming and/or antistatic substance, in particular of the quaternium or polyquaternium type, ("component (e)"), and
(f) at least one additive and/or at least one auxiliary substance ("component (f)"), in particular selected from the group formed by processing aids, stabilizers, emulsifying agents, dispersants, solubilizers, solvents, preservatives, antioxidants, humectants, thickening agents, viscosity regulators, antiseptics, colorants, flavourings, aromatic substances, fragrances, oils, perfumes and perfume oils, extenders, binders, wetting agents, vitamins, trace elements, minerals, micronutrients, care products, pH regulators and/or pH buffering systems, as well as combinations thereof,
wherein the composition contains the components (a), (b) and (c) in a proportion by weight of component (a) : component (b) : component (c) of 10-100 : 1-10 : 1-10.

11. Use of a composition as claimed in one of the preceding claims, for the treatment and/or care of keratinous fibres or substrates, in particular human hair.

12. Use as claimed in claim 11,
in connection with a hair colouring and/or hair tinting method, in particular wherein the composition is used during and/or after hair colouring or hair tinting; or else
for the protection of keratinous fibres or substrates, in particular human hair, from exogenous damage, in particular oxidative damage, in particular oxidative damage by hair colouring or hair tinting agents; or else
to improve the shine of keratinous fibres or substrates, in particular human hair, preferably to improve the shine of coloured or tinted keratinous fibres or substrates, in particular coloured or tinted human hair; or
to improve the wash resistance of keratinous fibres or substrates, in particular human hair, preferably to improve the wash resistance of coloured or tinted keratinous fibres or substrates, in particular coloured or tinted human hair; or else
to improve the combability of keratinous fibres or substrates, in particular human hair, preferably to improve the combability of coloured or tinted keratinous fibres or substrates, in particular coloured or tinted human hair; or else
to improve the static properties of keratinous fibres or substrates, in particular human hair, preferably to improve the static properties of coloured or tinted keratinous fibres or substrates, in particular coloured or tinted human hair; or else
to improve the feel and/or softness of keratinous fibres or substrates, in particular human hair, preferably to improve the feel and/or softness of coloured or tinted keratinous fibres or substrates, in particular coloured or tinted human hair; or else
to inhibit or reduce damage to keratinous fibres or substrates, in particular human hair, during (hair) colouring or (hair) tinting procedures, in particular during oxidative dyeing.

13. A hair treatment agent, in particular a hair care agent, preferably a hair colouring agent, hair tinting agent, hair washing agent, shampoo, hair rinse, conditioner, hair cream, hair lotion, hair oil, hair tonic, hair gel, hair wax or the like, containing a composition as claimed in one of the preceding claims.

14. A hair treatment unit, in particular to colour and/or tint keratinous fibres or substrates, in particular human hair, preferably in the form of a kit of parts,
wherein the hair treatment unit comprises, as separated and/or separate, in particular spatially separated but functionally connected and/or functionally arranged components:
(A) a first component, which contains at least one active substance for colouring and/or tinting keratinous fibres or substrates, in particular human hair, on the one hand; and
(B) a second component, which on the other hand contains a composition as claimed in one of the preceding claims.

15. A hair treatment unit as claimed in claim 14,
wherein the first component (A) contains, as the active substance for colouring and/or tinting keratinous fibres or substrates, in particular human hair, at least one substance from the group formed by oxidative dyes, oxidative dye precursors, oxidizing agents and direct dyes as well as combinations thereof, optionally together with excipients and auxiliary substances which are usual in hair colorants; and/or
wherein the oxidative dye precursors comprise at least one developer substance and at least one coupler substance; and/or
wherein the oxidizing agent is selected from the group formed by hydrogen peroxide, peroxides, perboric acid and perborates, as well as combinations thereof; and/or wherein the first component (A) and the second component (B) are manufactured and/or intended for mixing for use, in particular in the context of a method for colouring and/or tinting keratinous fibres or substrates; and/or
wherein the first component (A) and the second component (B) have been placed together and/or are packaged in joint outer packaging and/or a packaging unit, in particular in a joint multi-component packaging unit; in particular wherein the outer packaging and/or packaging unit, in particular the multi-component packaging unit, comprises a first container containing the first component (A) as well as a second container containing the second component (B); and/or in particular wherein the outer packaging and/or packaging unit, in particular the multi-component packaging unit, furthermore contains at least one further component (C), in particular selected from the group formed by (i) applicators such as paintbrushes, brushes, sponges, combs or the like, (ii) mixing aids, in particular stirring vessels and mixing or stirring aids, (iii) items of protective clothing, such as gloves, in particular disposable (protective) gloves, (iv) conditioners, (v) hair washing agents and shampoos, as well as (vi) instructions for handling, in particular instructions for use, and combinations thereof.

## Revendications

1. Composition, notamment composition, cosmétique, destinée de préférence au traitement et/ou au soin de fibres ou substrats kératiniques, notamment de cheveux humains,
la composition contenant, notamment en association et/ou en mélange,
(a) au moins un polymère à base de cystine (« composant (a) »), le polymère à base de cystine et/ou le composant (a) étant ou comprenant un copolymère cystine/siloxane (copolymère cystine/silicone),
(b) au moins une protéine et/ou un peptide de *Pisum sativum L.* (« composant (b) ») et
(c) au moins un extrait de *Selaginella lepidophylla* (fausse rose de Jéricho) (« composant (c) »)
la composition contenant les composants (a), (b) et (c) dans une proportion rapportée au poids du composant (a) : composant (b) : composant (c) de 10-100 : 1-10 : 1-10.

2. Composition selon la revendication 1,
la composition étant un produit de traitement capillaire, notamment un produit de soin capillaire, de préférence un produit de coloration capillaire, un produit de coloration capillaire semi-permanente, un bain capillaire, un shampooing, un après-shampooing, un baume (conditionneur), une crème capillaire, une lotion capillaire, une huile de soin capillaire, un tonique capillaire, un gel capillaire, une cire capillaire ou similaires et/ou la composition étant conçue et/ou préparée sous la forme de produit de traitement capillaire, notamment de produit de soin capillaire, de préférence de produit de coloration capillaire, de produit de coloration capillaire semi-permanente, de bain capillaire, de shampooing, d'après-shampooing, de baume (conditionneur), de crème capillaire, de lotion capillaire, d'huile de soin capillaire, de tonique capillaire, de gel capillaire, de cire capillaire ou similaires ou étant un composant de ces derniers.

3. Composition selon la revendication 1 ou 2,
la composition contenant par ailleurs au moins un support compatible du point de vue cosmétique (excipient) et/ou la composition contenant les ingrédients, notamment les composants (a), (b) et (c), ainsi que des ingrédients supplémentaires, présents le cas échéant, dans un support compatible du point de vue cosmétique (excipient) ou conjointement avec le support compatible du point de vue cosmétique (excipient).

4. Composition selon l'une quelconque des revendications précédentes,
le polymère à base de cystine et/ou le composant (a) présentant un poids moléculaire moyen en poids (Mw) de l'ordre de 5.000 g/mole à 1.000.000 g/mole, notamment de l'ordre de 7.500 g/mole à 750.000 g/mole, de préférence de l'ordre de 10.000 g/mole à 500.000 g/mole, de manière particulièrement préférentielle de l'ordre de 12.000 g/mole à 100.000 g/mole, de manière tout particulièrement préférentielle, de l'ordre de 15.000 g/mole à 50.000 g/mole, de manière encore plus préférentielle, de l'ordre de 17.500 g/mole à 30.000 g/mole ; notamment le poids moléculaire moyen en poids (Mw) étant déterminé par chromatographie par perméation de gel (GPC), notamment selon la norme DIN 55671, de préférence en utilisant un étalon de polystyrène ; et/ou
le polymère à base de cystine et/ou le composant (a) contenant de la cystine en quantités rapportées au poids, rapportées au polymère à base de cystine et/ou au composant (a) de l'ordre de 10 à 80 % en poids, notamment de l'ordre de 15 à 70 % en poids, de préférence de l'ordre de 20 à 60 % en poids, de manière particulièrement préférentielle de l'ordre de 25 à 50 % en poids ; et/ou
le polymère à base de cystine et/ou le composant (a) comprenant ou étant un copolymère, le copolymère comprenant une ou plusieurs unités, chaque unité comprenant un radical de cystine, le radical de cystine étant chaque fois lié à un composant contenant du silicium, le composant contenant du silicium comprenant au moins une liaison siloxane ; et/ou
le polymère à base de cystine et/ou le composant (a) étant ou comprenant un copolymère cystine/siloxane (copolymère cystine/silicone) de type poly[cystindiyl-bis-(polyglycol-propylsilane-triol)], notamment de type poly[cystindiyl-bis-(polyéthylène-glycol-propylsilantriol)] ou de type poly[cystindiyl-bis-(polypropylèneglycol-propylsilantriol)].

5. Composition selon l'une quelconque des revendications précédentes,
le polymère à base de cystine et/ou le composant (a) étant ou comprenant un polymère, lequel présente la structure suivante :
HO-[Si(OH)₂-X-cystine-X-Si(OH)₂-O]-H
le radical X représentant un groupe de composés (lieurs) notamment un groupe de composés (lieurs) résultant de la réaction d'un groupe amino sur la cystine avec un groupe amino réactif sur le groupe contenant du silicium, de préférence un groupe de composés (lieurs) de la formule (CH₂)₃-O-CH₂-CH(OH)CH₂, et n représentant un nombre de 1 à 200 incluant les limites supérieures et inférieures ;
notamment le polymère à base de cystine contenant de la cystine dans des quantités rapportées au poids, rapportées au polymère à base de cystine, de l'ordre de 10 à 80 % en poids, notamment de l'ordre de 15 à 70 % en poids, de préférence de l'ordre de 20 à 60 % en poids, de manière particulièrement préférentielle de l'ordre de 25 à 50 % en poids ; et/ou
notamment le polymère à base de cystine présentant un poids moléculaire (Mw) moyen en poids de l'ordre de 5.000 g/mole à 1.000.000 g/mole, notamment de l'ordre de 7.500 g/mole à 750.000 g/mole, de préférence de l'ordre de 10.000 g/mole à 500.000 g/mole, de manière particulièrement préférentielle de l'ordre de 12.000 g/mole à 100.000 g/mole, de manière tout particulièrement préférentielle de l'ordre de 15.000 g/mole à 50.000 g/mole, de manière encore plus préférentielle de l'ordre de 17.500 g/mole à 30.000 g/mole ; notamment le poids moléculaire (Mw) moyen en poids étant déterminé par chromatographie par perméation de gel (GPC), notamment selon la norme DIN 55671, de préférence en utilisant un étalon de polystyrène.

6. Composition selon l'une quelconque des revendications précédentes,
la composition contenant en tant que composant (b) au moins un peptide de *Pisum sativum L*., et/ou au moins une protéine hydrolysée de *Pisum sativum L.*

7. Composition selon l'une quelconque des revendications précédentes,
la composition contenant le polymère à base de cystine et/ou le composant (a) dans des quantités de l'ordre de 0,01 à 50 % en poids, notamment de l'ordre de 0,05 à 25 % en poids, de préférence de l'ordre de 0,1 à 20 % en poids, de manière particulièrement préférentielle de l'ordre de 0,5 à 15 % en poids, de manière encore plus préférentielle de l'ordre de 1 à 10 % en poids, rapportées à la composition ; et/ou
la composition contenant l'au moins une protéine et/ou un peptide de *Pisum sativum* et/ou le composant (b) dans des quantités de l'ordre de 0,0001 à 25 % en poids, notamment de l'ordre de 0,001 à 20 % en poids, de préférence de l'ordre de 0,005 à 15 % en poids, de manière particulièrement préférentielle de l'ordre de 0,01 à 10 % en poids, de manière encore plus préférentielle de l'ordre de 0,02 à 5 % en poids, rapportées à la composition ; et/ou
la composition contenant l'au moins un extrait de *Selaginella lepidophylla* (fausse rose de Jéricho) et/ou le composant (c) dans des quantités de l'ordre de 0,0001 à 25 % en poids, notamment de l'ordre de 0,001 à 20 % en poids, de préférence de l'ordre de 0,005 à 15 % en poids, de manière particulièrement préférentielle de l'ordre de 0,01 à 10 % en poids, de manière encore plus préférentielle de l'ordre de 0,02 à 5 % en poids, rapportées à la composition.

8. Composition selon l'une quelconque des revendications précédentes,
la composition étant réglée à une valeur pH supérieure à 5, notamment supérieure à 5,5 et/ou la composition étant réglée à une valeur pH légèrement acide à alcaline, notamment à une valeur pH de l'ordre de 5,5 à 11,0.

9. Composition selon l'une quelconque des revendications précédentes,
la composition contenant par ailleurs (d) au moins un filtrat de ferment de racine de radis, notamment un filtrat de ferment de racine de radis et de leuconostoc, susceptible d'être obtenu notamment par fermentation de racines de *Raphanus-sativus* à l'aide du micro-organisme *Leuconostoc*, (« composant (d) ») ; notamment la composition contenant le filtrat de ferment de racine de radis et/ou le composant (d) dans des quantités de l'ordre de 0,00001 à 20 % en poids, notamment de l'ordre de 0,0001 à 10 % en poids, de préférence de l'ordre de 0,0005 à 5 % en poids, de manière particulièrement préférentielle de l'ordre de 0,001 à 2 % en poids, rapportées composition ; et/ou
la composition contenant par ailleurs (e) au moins un principe actif filmogène et/ou antistatique, notamment de type quaternium ou polyquaternium (« composant (e) ») ; notamment la composition contenant le principe actif filmogène et/ou antistatique et/ou le composant (e) dans des quantités de l'ordre de 0,001 à 40 % en poids, notamment de l'ordre de 0,01 à 30 % en poids, de préférence de l'ordre de 0,1 à 25 % en poids, de manière particulièrement préférentielle de l'ordre de 0,5 à 20 % en poids, rapportées à la composition ; et/ou
la composition contenant par ailleurs (f) au moins un additif et/ou au moins un adjuvant (« composant (f) »), notamment choisi dans le groupe comprenant les adjuvants technologiques, les agents stabilisateurs, les agents émulsifiants, les agents dispersants, les agents de solubilisation, les solvants, les conservateurs, les antioxydants, les agents humectants, les agents épaississants, les régulateurs de viscosité, les antiseptiques, les colorants, les agents aromatiques, les agents odorants, les fragrances, les huiles, les parfums et les huiles parfumées, les extendeurs, les agents liants, les agents réticulants, les vitamines, les oligo-éléments, les minéraux, les micro-nutriments, les agents de traitement, les ajusteurs de pH et/ou les systèmes tampons pH, ainsi que leurs associations.

10. Composition, notamment composition cosmétique, destinée de préférence au traitement et/ou au soin de fibres ou substrats kératiniques, notamment de cheveux humains, notamment composition selon l'une quelconque des revendications précédentes,
la composition contenant, notamment en association et/ou en mélange,
(a) au moins un polymère à base de cystine (« composant (a) »), le polymère à base de cystine et/ou le composant (a) étant ou comprenant un copolymère cystine/siloxane (copolymère cystine/silicone),
(b) au moins une protéine et/ou un peptide de *Pisum sativum L.* (« composant (b) ») et
(c) au moins un extrait de *Selaginella lepidophylla* (fausse rose de Jéricho) (« composant (c) »),
(d) au moins un filtrat de ferment de racine de radis, notamment un filtrat de ferment de racine de radis et de leuconostoc, susceptible d'être obtenu notamment par fermentation de racines de *Raphanus-sativus* à l'aide du micro-organisme *Leuconostoc* (« composant (d) »),
(e) au moins un principe actif filmogène et/ou antistatique, notamment de type quaternium ou polyquaternium, (« composant (e) ») et
(f) au moins un additif et/ou au moins un adjuvant (« composant (f) »), notamment choisi dans le groupe comprenant les adjuvants technologiques, les agents stabilisateurs, les agents émulsifiants, les agents dispersants, les agents de solubilisation, les solvants, les conservateurs, les antioxydants, les agents humectants, les agents épaississants, les régulateurs de viscosité, les antiseptiques, les colorants, les agents aromatiques, les agents odorants, les fragrances, les huiles, les parfums et les huiles parfumées, les extendeurs, les agents liants, les agents réticulants, les vitamines, les oligo-éléments, les minéraux, les micro-nutriments, les agents de traitement, les ajusteurs de pH et/ou les systèmes tampons pH, ainsi que leurs associations,
la composition contenant les composants (a), (b) et (c) dans une proportion rapportée au poids du composant (a) : composant (b) : composant (c) de 10-100 : 1-10 : 1-10.

11. Utilisation d'une composition selon l'une quelconque des revendications précédentes pour le traitement et/ou le soin de fibres ou substrats kératiniques, notamment de cheveux humains.

12. Utilisation selon la revendication 11
en relation avec un procédé de coloration capillaire ou de coloration capillaire semi-permanente, notamment la composition étant utilisée pendant et/ou après la coloration ou la coloration semi-permanente de cheveux ; ou encore
pour la protection de fibres ou substrats kératiniques, notamment de cheveux humains contre des détériorations exogènes, notamment des détériorations oxydatives, notamment des détériorations oxydatives dues à des produits de coloration capillaire ou des produits de coloration capillaire semi-permanente ; ou encore
pour l'amélioration de la brillance de fibres ou substrats kératiniques, notamment de cheveux humains, de préférence pour l'amélioration des fibres ou substrats kératiniques ayant subi une coloration ou une coloration semi-permanente, notamment de cheveux humains ayant subi une coloration ou une coloration semi-permanente ; ou
pour l'amélioration de la résistance au lavage de fibres ou substrats kératiniques, notamment de cheveux humains, de préférence pour l'amélioration de la résistance au lavage des fibres ou substrats kératiniques ayant subi une coloration ou une coloration semi-permanente, notamment de cheveux humains ayant subi une coloration ou une coloration semi-permanente ; ou encore
pour l'amélioration du démêlage de fibres ou substrats kératiniques, notamment de cheveux humains, de préférence pour l'amélioration du démêlage des fibres ou substrats kératiniques ayant subi une coloration ou une coloration semi-permanente, notamment de cheveux humains ayant subi une coloration ou une coloration semi-permanente ; ou encore
pour l'amélioration des propriétés statiques de fibres ou substrats kératiniques, notamment de cheveux humains, de préférence pour l'amélioration des propriétés statiques des fibres ou substrats kératiniques ayant subi une coloration ou une coloration semi-permanente, notamment de cheveux humains ayant subi une coloration ou une coloration semi-permanente ; ou encore
pour l'amélioration de la texture et/ou de la souplesse de fibres ou substrats kératiniques, notamment de cheveux humains, de préférence des fibres ou substrats kératiniques ayant subi une coloration ou une coloration semi-permanente, notamment de cheveux humains ayant subi une coloration ou une coloration semi-permanente ; ou encore pour éviter ou restreindre la détérioration de fibres ou substrats kératiniques, notamment de cheveux humains, notamment lors de procédé de coloration (capillaire) ou de coloration (capillaire) semi-permanente, notamment lors du changement de couleur par oxydation.

13. Produit de traitement capillaire, notamment produit de soin capillaire, de préférence produit de coloration capillaire, produit de coloration capillaire semi-permanente, bain capillaire, shampooing, après-shampooing, baume (conditionneur), crème capillaire, lotion capillaire, huile de soin capillaire, tonique capillaire, gel capillaire, cire capillaire ou similaires, contenant une composition selon l'une quelconque des revendications précédentes.

14. Unité de traitement capillaire, notamment destinée à la coloration et/ou à la coloration semi-permanente de fibres ou substrats kératiniques, notamment de cheveux humains, de préférence sous la forme d'un kit (Kit-of-parts),
l'unité de traitement capillaire comportant sous la forme de composants distincts et/ou séparés, notamment physiquement séparés, mais en cohérence fonctionnelle et/ou en association mutuelle fonctionnelle :
(A) un premier composant, lequel contient au moins un principe actif destiné à la coloration et/ou à la coloration semi-permanente de fibres ou substrats kératiniques, notamment de cheveux humains, d'une part ; et
(B) un deuxième composant, lequel contient une composition selon l'une quelconque des revendications précédentes, d'autre part.

15. Unité de traitement capillaire selon la revendication 14,
le premier composant (A) contenant en tant que principe actif destiné à la coloration et/ou à la coloration semi-permanente de fibres ou substrats kératiniques, notamment de cheveux humains au moins un principe actif du groupe comprenant des colorants d'oxydation, des précurseurs de colorants d'oxydation, des agents oxydants et des colorants directs, ainsi que leurs associations, le cas échéant, conjointement avec des excipients et des adjuvants habituels dans des produits de coloration capillaire ; et/ou
les précurseurs de colorants d'oxydation comprenant au moins un développateur et au moins une substance de couplage ; et/ou
le produit oxydant étant choisi dans le groupe comprenant le peroxyde d'hydrogène, des peroxydes, l'acide perborique et des perborates, ainsi que leurs associations ; et/ou
le premier composant (A) et le deuxième composant (B) étant préparés pour et/ou destinés à être mélangés dans le cas d'application, notamment dans le cadre d'un procédé de coloration et/ou de coloration semi-permanente de fibres ou substrats kératiniques ; et/ou
le premier composant (A) et le deuxième composant (B) étant réunis et/ou emballés dans un suremballage et/ou une unité d'emballage commun(e), notamment dans une unité d'emballage multicomposants commune ; notamment le suremballage et/ou l'unité d'emballage, notamment l'unité d'emballage multicomposants comprenant un premier réceptacle (contenant) qui contient le premier composant (A), ainsi qu'un deuxième réceptacle (contenant) qui contient le deuxième composant (B) ; et/ou notamment le suremballage et/ou l'unité d'emballage, notamment l'unité d'emballage multicomposants contenant par ailleurs au moins un composant (C) supplémentaire, choisi notamment dans le groupe comprenant (i) les outils d'application, comme les pinceaux, les brosses, les éponges, les peignes ou similaires, (ii), les outils de mélange, comme les récipients de mélanges et les outils de mélange ou de brassage, (iii), les accessoires vestimentaires protecteurs, comme des gants, notamment des gants (protecteurs) jetables, (iv) les baumes (conditionneurs), (v) les bains capillaires et shampooings, ainsi que (v) les instructions de manipulation, notamment les modes d'emploi et leurs associations.
